# EUROPEAN PATENT SPECIFICATION

(11) **EP 1 399 077 B1**
(45) Date of publication and mention of the grant of the patent: **23.08.2006**
(21) Application number: 02706259.5
(22) Date of filing: 13.02.2002
(51) Int. Cl.: A61B 17/70

(54) **Intervertebral disc repair compression device and trocar**
Kompressionsvorrichtung und Trokar zum Reparieren einer Zwischenwirbelprothese
Dispositif de compression et trocar pour réparation de disque vertébral

(30) Priority: 13.02.2001 US 268666 P; 11.06.2001 US 297556 P; 03.08.2001 US 310131 P; 26.09.2001 US 325111 P; 17.10.2001 US 330260 P
(43) Date of publication of application: 24.03.2004
(73) Proprietor: Yeung, Jeffrey E., San Jose, CA 95127 (US); Yeung, Teresa, T., San Jose, CA 95127 (US)
(72) Inventor: Yeung, Jeffrey E., San Jose, CA 95127 (US); Yeung, Teresa, T., San Jose, CA 95127 (US)
(74) Representative: Stainthorpe, Vanessa Juliet
(86) International application number: PCT/US2002/004301
(87) International publication number: WO 2002/064044

(56) References cited:
- WO-A-00/40159
- WO-A-01/95818
- DE-A- 4 440 346
- FR-A- 2 586 183

## Description

### FIELD OF INVENTION

This invention relates to devices for treating disc protrusion, segmental instability, spinal stenosis, scoliosis or kyphosis by compressing or thickening the intervertebral disc. The invention also proposes a device to promote annular regeneration and adhesion onto the end plate to accelerate healing of the dysfunctional disc and spondylolisthesis.

### BACKGROUND, EXISTING SURGICAL PRACTICES AND PRIOR INVENTIONS

Low-back pain is one of the most prevalent, costly and debilitating ailments afflicting mankind. Seventy to eighty-five percent of all people have back pain at some time in their life. Symptoms are most common among middle-aged adults and are equally common among both men and women. Back pain related to disc disorders, however, is more prevalent among men. The recurrence rate of low back pain ranges from 20% to 44% annually, with lifetime recurrences of 85% (National Institute of Health Guide, Vol. 26, 16, May 16, 1997).

Low back pain is very costly to patients, our health care system and society. For many, no position can ease their pain or numbness, not even bed rest. It is often the reason for decreased productivity due to loss of work hours, addiction to pain-killing drugs, emotional distress, prolonged hospital stays, loss of independent living, unplanned early retirements and even financial ruin. Each year in the US, about 2% of the work force have back injuries covered by worker's compensation, with about $12 billion spent directly on medical costs in 1994.

### Bulging or Herniated Intervertebral Discs

Most back pain is initiated with a defective or damaged intervertebral disc. The disc is comprised of nucleus pulposus and annulus. The nucleus pulposus is highly gelatinous with a composition of 70-90% water, 25-60% proteoglycan (dry weight) and 10-20% collagen (dry weight). The function of the nucleus pulposus is to sustain prolonged compression during the day and to resiliently re-inflate and reestablish disc height during the night. The pulposus is retained and surrounded by layers of cartilaginous annulus. Together the pulposus and the annulus behave as a resilient cushion. In the erect position, the weight of the body constantly compresses upon a stack of these cushions alternating between a series of vertebrae. During constant compression, the pulposus in each disc also behaves as a water reservoir, which is slowly and constantly being squeezed and drained of its water content through the end plates connected to the vertebrae. As a result, the disc height decreases throughout the day.. During bed rest, the weight of the body no longer compresses the disc. Due to the water absorbing nature of the nucleus pulposus, the flow of water then reverses from the vascular vertebrae back into the proteoglycan and collagen. As a result, the disc height is reestablished and ready to provide support for another day.

With aging and degeneration, the viscoelastic property of the nucleus pulposus undergoes a transition from fluid-like to solid-like behavior (J.C. latridis et. al., Journal of Orthopaedic Research, 15:318-322, 1997). Under dynamic conditions, the gelatinous nucleus pulposus exhibits predominantly solid-like behavior with values for dynamic modulus ranging from 7 to 20 kPa (J.C. Iatridis et. al., J. Biomechanics, Vol. 30, No. 10, 1005-1013, 1997). As a result, both the resiliency and disc height diminish.

Bulges are most commonly reported at the posterior-lateral regions of the discs. The bulging regions are commonly divided into zones. The posterior region where the spinal cord is located is called the central zone. Adjacent to both sides of the central zone are the entrance zones, followed by pedicle zones, the exit zones, and the far lateral zones. Bulges at the far lateral zones, the most accessible area, have the highest surgical success rate.

Some causes that contribute to low back pain are classified. Type I: Acute back sprain involves damage to ligaments, muscles or even the vertebral end plates from physical overload. Type II: Organic idiopathic spine pain occurs from increased fluid uptake by the disc. Type III: Disruption of posteriolateral annular fibers irritates nerves associated with the sacroiliac region, buttock and the back of the thigh. This situation may resolve itself through reabsorption or neutralization by phagocytosis of the disrupted annular fibers. Type IV: Nerve root irritation by the bulging disc leads to sciatica. This type of disc protrusion is traditionally repaired surgically by tissue removal, chemonucleolysis or percutaneous discectomy. Type V: Nerve irritation by wandering sequestered disc material has unpredictable exacerbation and remission. Type VI: Sequestrum of the annulus and/or nucleus into the spinal canal or intervertebral foramen results in nerve irritation from inflammation, mechanical pressure, chemical irritation, autoimmune response or combinations of irritants. Type VII - A degenerated disc, with substantial decrease in mechanical properties, is often associated with pain and disability.

The most common reason for recurrent pain is the bulging or herniation of an intervertebral disc. The traditional surgical treatment for a bulging or herniated disc is a series of tissue removing, filling and supporting procedures: (1) laminectomy, excision of the posterior arch of a vertebra which covers part of the herniated disc, (2) discectomy, removal of the disc, (3) bone harvesting usually from the patient's iliac crest, (4) donor bone packing into the vacant disc space, (5) supporting adjacent vertebral bodies with rods, connectors, wire and screws, (6) bone cement filling the donor site, and finally (7) closing multiple surgical sites.

Numerous postoperative complications can occur after a back surgery. The major ones are lumbar scarring and vertebral instability. The scar tissue extends and encroaches upon the laminectomy site and intervertebral foramen, then once again, pain returns, which leads to more surgery. In fact, repeat operations are very common, 10-20%. Unfortunately, the success rates of repeat operations are often less, in some cases, far less than the first. More operations lead to more scarring and more pain. Current recommendations to the patients are to avoid surgical procedures unless the pain and inconveniences are absolutely unbearable. Even for the fortunate patients with long term success following discectomies performed twenty years ago, their isokinetic test results clearly indicate weaknesses compared to populations without discectomies.

There was and still is increasing interest in more effective and less invasive surgical techniques on the spine to reduce both trauma and cost. The major objectives of surgery on bulging or herniated lumbar discs are (1) decompression of the involved nerve root or roots, and (2) preservation of bony spine, joints and ligaments.

Chymopapain is an enzyme used to digest the nucleus pulposus, the viscous and gel-like substance in the central portion of the disc, which then creates space for the bulging part of the disc to pull back from the encroached nerve root. The needle for injecting the chymopapain is accurately guided to the mid-portion of the disc by a stereotaxic device. The overall success rate is documented as high as 76%. However, some patients are allergic to the treatment and die from anaphylaxis. Some suffer from serious neuralgic complications, including paraplegia, paresis, cerebral hemorrhage and transverse myelitis.

Percutaneous nuclectomy is an alternative method for removing nucleus pulposus without the allergic reaction of chymopapain, and it rarely causes epidural scarring. Similar to the chymopapain injection, a needle followed by a tube-like instrument is guided and confirmed by anteroposterior and lateral fluoroscopy. The nucleus pulposus is then removed mechanically or by vacuum. As a result, a void is created within the disc and the bulging decreases, like the air being released from a worn out tire, with the hope that the bulging portion of the disc will recede and no longer encroach upon the adjacent nerve root. This type of procedure is often referred to as one of the decompression procedures. However, the amount of nucleus pulposus removed has been documented to be insignificantly small, with unpredictable results and a low rate of success.

Recently, several devices (US Patent No. 5,800,550 to Sertich, 1998; US Patent No. 5,683,394 to Rinner, 1997; US Patent No. 5,423,817 to Lin, 1995; US Patent No. 5,026,373 to Ray et. al., 1991) were designed to fortify the disc space between vertebrae. These types of devices are frequently referred to as spinal cages. Before inserting the device into the disc, the affected disc with portions of vertebral bone above and below the disc are cored out. Usually two holes are cored on each side of the disc for insertion of two spinal cages. Donor bone or bone growth promoting substances are packed into the porous cages. As the vertebrae heal from the coring, new bone grows into and permanently secures the porous cages. The purpose of using spinal cages is to replace the disc and keep the vertebrae apart. However, these vertebrae are permanently fused to each other, without resilient cushion, rotation or mobility.

An improved version of a metallic spinal fusion implant (US patent 5,782,832 to Larsen and Shikhman, 1998) tries to provide both rotational and cushioning capabilities. This invention resembles a disc prosthesis following a complete discectomy. Therefore, at the least, all the complications and postsurgical problems associated with a discectomy also apply when this device is used.

Patent application WO 00/40159 by Yeung et al., on whose disclosure the preamble of claim 1 is based, introduces some devices and methods for some devices and methods for fastening herniated and/or bulging discs. The application covers a resiliently bent fastener, screw, suture, staple and tack, with methods to fasten and hold in the bulging annulus. Another patent application, WO 01/95818, by Yeung, introduces more devices and methods for fastening the intervertebral disc to treat nerve impingement, vertebral instability and spinal stenosis.

### Spinal Stenosis

Disc degeneration has been shown to be the first stage in the aging processes of the spine. As the process develops, the circumferential and radial tears of the annulus become evident, proteoglycan and collagen dehydrates (water content of nucleus pulposus fall from 85% to 70%), resulting in decreased disc height. As the annulus continues to degenerate, the disc bulges and/or flattens, narrowing the central canal. The condition is called spinal stenosis. Spinal stenosis is a progressive and dynamic process. Depending on the amount and location of the stenosis, the symptoms may be restricted to a single isolated root, as in lateral recess stenosis, or may involve multiple levels. A normal lumbar canal has a 12-mm or greater anterior-posterior diameter. However, the nerve root within the small neuroforamen is particularly susceptible to impingement from a lateral bulging disc and is often further aggravated by facet joint erosion or alteration.

Mechanical compression of spinal nerve roots from spinal stenosis has a variety of clinical symptoms, including weakness, reflex alterations, pain and paresthesias. Intermittent neurogenic claudication (limping) has been found in patients with stenosis. Clinical features include low back pain and dysesthesia (sense impairment) spreading diffusely down the posteriolateral parts of the lower extremities, often asymmetrically. Pain is typical and often exacerbated by walking and standing. Symptoms disappear with sitting, recumbency or other changes in posture that reverse the lumbar lordosis (curvature). To distinguish clinically between spinal stenosis and herniated disc, restriction of straight-leg raising is frequently not painful in patients with spinal stenosis, but painful in patients with disc herniation. Spinal stenosis complicated by a herniated disc and spondylosis was noted to occur in 39% of 227 patients with low back pain Spinal stenosis was the only cause of symptoms in only 8% of patients (M. Camins. et. aL, The Lumbar Spine, Raven Pres, NY, 1987, pp.149).

As the disc space narrows, the settling of the facet joints greatly increases mechanical stress, leading to joint erosion. As the joint erodes, the narrowed space of the neuroforamen diminishes. The nerve root is entrapped and surrounded by the pedicle (the bony extension forming the facet joint) superiorly, the bulging disc inferiorly, the vertebral body osteophytes anteriorly and the hypertrophied degenerative facets posteriorly. Most nerve entrapment occurs in the vicinity of the pedicle. This has been referred to as the hidden zone. The nerve root and ganglion are highly protected and covered by bone. Decompression of the nerve root using current surgical technique requires a significant amount of bone and disc removal, making the procedure very invasive. Nerve root impingement at the extraforaminal zone is usually from ligament, lateral disc herniation or tumor.

Although the majority of lumbar spinal conditions should initially be treated conservatively, certain conditions do require urgent surgical intervention. Significant or progressive weakness of the lower extremity in the form of either footdrop or the inability to toe stand may result in irreversible damage. It is imperative to initiate early diagnostic evaluation followed by prompt surgical treatment.

Decompression laminectomy (excision of the posterior arch of a vertebra) is the standard procedure advocated. The ligamentum flavum is usually left intact to protect the dura, and the facet joints are protected. But in certain instances less aggressive laminotomies (removal of a portion of lamina) may be appropriate with hospitalization 5 to 7 days postoperatively. Ambulation may begin within 24 hours after surgery and often on the same day. Despite the invasiveness of the procedure, mortality rate is low (0.1 - 0.6%). Other complications include neurologic deficit, temporary in 5%, permanent deficit in 1.3%, cerebrospinal fluid fistulas (leakage) 4.6%, infection 0.5% - 8.5%, reoperation 9.8% and increased risk of facet fractures.

A 20-year follow-up study, noted complete relief of preoperative signs and symptoms in 68% of patients: The remaining patients (32%) continue having lumbago (pain in low back and buttocks), intermittent claudication (lameness), motor deficit, sciatica (pain radiating from the back into lower extremity), paraplegia (paralysis of the legs) and/or micturition (the passage of urine).

### Segmental Instability

Instability across the motion segment (vertebral body-disc-vertebral body) can occur as the disc degenerates. Segmental instability resembles an out-of-control car riding on one or more flat tires with deflated and unsupported sidewalls. A flattened intervertebral disc causes excessive movement between vertebral bodies, leading to pain in surrounding ligaments and facet joints. Depletion of nucleus pulposus from the percutaneous nuclectomy procedure can accelerate disc flattening or thinning, leading to segmental instability and/or spinal stenosis. Although it might not be grossly detected radiographically, this instability is most apparent during compressional or rotational movements. Under normal conditions, the spinal motion segment and particularly the neuroforamen can smoothly and symmetrically accommodate rotational motions, as well as flexion and extension, without significant alteration of available space. However, as the disc degenerates, the ligaments buckle, the facet joints mal-align and unstable movement appears during routine vertebral motions. With narrowing of the central canal and neuroforamen, unstable vertebral movements produce irritation, inflammation and pain.

Treatment recommended for segmental instability is mostly rest and drug therapy, including analgesics, anti-inflammatory agents, oral steroids, muscle relaxants and antidepressants.

### Spondylolisthesis

The axial compression force upon the L5-S1 level is between 1500 and 2500 N, bending moment between 15 NM and 25 NM. Due to the curvature of the spine, approximately 20% of the axial compression force is a forward-directed shear force. (Bergmark A., Acta Orthop Scand Suppl:230-238, 1989). As the shear force works on an aging and degenerating disc, the forward sliding process begins. The shear force intensifies as the L5 moves forward and provides more and more leverage. Finally, the ventral (forward) sliding of L5 in relation to S1, called spondylolisthesis, brings a great deal of pain from many possible nerve impingements, including impingement by the transverse process and ligament.

When slippage is less than 50%, vertebral traction alone can usually reposition the L5-S1 disc without removing the L5-S1 disc. Lumbosacral fusion is followed. However, if the slippage is greater than 50%, additional instrumentation may be required to reposition the L5. During the repositioning process, the L5-S1 disc may not be spared. Lumbosacral fusion is necessary and usually done with pedicle screws and instrumentation in an open surgery.

### Deformities of Spine

Most spine deformities are innate. Surgical correction of these deformities is highly invasive and many require repeat surgeries due to instrumentation fatigue/failure or complications. Scoliosis is a condition involving lateral curves or angular deviations of one or more vertebral segments. Commonly known as humpback, kyphosis is an exaggeration of the posterior convexity of the thoracic vertebral column. Three common causes of kyphosis are (1) absence of T-12 vertebral body, (2) malformation and incomplete segmentation of vertebral body, and (3) indentation of anterior portion of vertebral body from compression. Lordosis is an exaggeration of the posterior concavity of the spine characteristic of the lumbar region. Commonly known as swayback, it indicates extreme anterior curvature of the lumbar spine.

### SUMMARY OF INVENTION

The majority of back pain can be traced to one or more degenerative or damaged discs. Instead of repairing the disc, current surgical devices and techniques are designed to decompress nerve impingement by removing adjacent tissues, and/or fusing the spine with instruments. The common problems associated with the techniques are scarring, instrument fatigue/failure and/or progressive degeneration of the spine.

According to the invention there is provided a compression device according to the appendant claims.

In embodiments of the present invention, a saddle-shaped compressor with an annular contact surface thickening into a sloped surface is used to (1) compress the disc protrusion to alleviate nerve impingement, (2) fortify the bulging annulus to minimize segmental instability, (3) wedge into and thicken the disc to repair spinal stenosis, scoliosis, kyphosis or lordosis, and/or (4) atrophy the sinuvertebral nerve to treat discogenic pain. The disc-compressing compressor can be fastened (1) around the disc as a resilient clamp, (2) from a bracket anchored on the vertebral body, (3) through the disc with a bolt, or (4) through a portion of the disc and the end plate into the vertebral body with a screw.

Annular tissue is slow to heal. To facilitate the healing process, bleeding sites are surgically inflicted on the end plate with a straight or curved trocar. Oozing of the bleeding sites forms adhesion between the compressed annular tissue and the end plate, keeping the annular tissue from bulging out. The adhesion assists the fastened compressor in maintaining annular compression. The adhesion from surgically inflicted bleeding sites may be particularly useful in treating spondylolisthesis after the detached vertebral body has been realigned with the disc. The end plate bleeding sites can also serve as passages or channels to transport nutrients and metabolites between the vascular vertebral body and the avascular annulus, expediting healing or regeneration of the degenerative disc.

Discogenic pain is believed to originate from ingrowth of sinuvertebral nerves into a degenerative disc. Continual compression of the compressor over the nerve on the surface of the disc can atrophy the nerve, ceasing the transmission of the pain signal sensed within the degenerative disc.

The compressor can be elastically fastened to continuously compress into the disc. With time, the sloped surface of the compressor slowly wedges into the annulus to expand and thicken the disc. The annular expansion or thickening is maintained by plateau surfaces of the compressor shimmed between the epiphyses of vertebral bodies, thus elevating the disc height to alleviate nerve impingement from spinal stenosis. Similarly, one side of a disc can be selectively shimmed and elevated by elastic compression of the compressor to straighten and correct spinal deformities, such as scoliosis, kyphosis or lordosis with time.

### REFERENCE NUMBER

- 100: Intervertebral disc
- 101: Tightening elements
- 102: Nerve
- 103: Trocar
- 104: Sleeve with windows
- 105: End-plate
- 106: Slit opening
- 107: Strut
- 108: Head of sleeve with window
- 109: Thread
- 110: Hole for screw or bolt
- 111: Disc compressor
- 112: Indented portion
- 115: Epiphysis
- 116: Bolt head
- 117: Stabilizer lumen
- 118: End of lift spring
- 119: Annulus contact surface
- 120: Hole for bolt
- 121: Lift spring
- 122: Supporting plate
- 123: Spinal cord
- 124: Delivery device
- 125: Coil spring
- 126: Pivoting means
- 127: Elastic fastening means
- 128: Nucleus pulposus
- 129: Facet joint
- 130: Tip of the compressor
- 131: Delivery capsule
- 132: Latch
- 133: Socket drive
- 134: Stabilizer
- 135: Lip of stabilizer
- 139: Bracket
- 140: Sacrum
- 142: Superior articular process
- 143: Inferior articular process
- 159: Vertebral body
- 160: Tissue ingrowth opening
- 161: Bolt
- 162: Nut
- 163: Washer
- 164: Indentation
- 165: Slit hole for bolt or screw
- 167: Anterior longitudinal ligament
- 170: Sloped surface
- 171: Plateau surface
- 172: Pivotal peg or screw
- 173: Stop
- 176: Widening tool
- 177: Clamp grabber
- 178: Lock screw of widening tool
- 179: Lock wheel of widening tool
- 180: Hinge of lock screw
- 181: Handle of widening tool
- 182: Pivotal joint of widening tool
- 183: Lock slot
- 184: Impingement of nerve
- 185: Trocar guide
- 187: Screw
- 188: Casing of compressor
- 194: Ventral/dorsal ramus nerve root
- 195: Posterior longitudinal ligament
- 196: Nerve shield
- 198: Clamp
- 199: Widening mount
- 201: Support mount
- 202: Trough on shield
- 212: Strap
- 213: Distal tip of the nerve shield
- 214: Open channel of nerve shield
- 215: Staple
- 216: Sinuvertebral nerve
- 217: Screw entry
- 218: Biodegradable sleeve
- 220: Trocar sleeve
- 221: Label showing direction of curved trocar
- 223: Trough or indentation of compressor
- 224: Bleeding sites
- 225: Lumen of sleeve with window
- 226: Screw head
- 228: Opening for socket or screw driver
- 229: Locking mechanism
- 230: Dilator
- 231: Indentation of disc clamp
- 233: Outer surface
- 234: Spinal fusion

### BRIEF DESCRIPTION OF THE DRAWINGS

Figure 1 depicts a common disc **100** protrusion at or near the neuroforamen, impinging upon the ventral/dorsal ramus nerve root **194.**
Figure 2 shows a nerve shield **196** with a thin but blunt distal tip **213** to reach into or near the neuroforamen and a trough **202** to protect the nerve exiting from the neuroforamen.
Figure 3 indicates the nerve shield **196** reaching into or near the neuroforamen by sliding over the bulging annulus of the disc **100.**
**Figure 4** depicts two nerve shields **196** protecting the nerves **194** from instrumentation.
**Figure 5** shows an elastic clamp **198** comprising two disc compressors **111** with annular contact surfaces **119,** sloped surfaces **170,** plateau surfaces **171,** stops **173,** widening **199** and support **201** mounts.
Figure 6 shows a clamp-widening tool **176** equipped with clamp grabbers **177** and a locking mechanism capable of slow release.
Figure 7 depicts widening and placement of the disc clamp **198** by the widening tool **176** around the protruded disc **100.**
Figure 8 shows alleviation of nerve **194** impingement by clamping of the bulging annulus with compressors **111.** The size of the clamp/compressors **198/111** is enlarged disproportionately to the disc **100,** for clarification.
Figure 9 indicates the locations of compression by the compressors **111.** The important compressions are at area C and I, common locations of disc **100** protrusion.
Figure 10 indicates an elastic strap **212** threaded through the support mount **201** to support the disc clamp **198.** The elastic strap **212** is secured by a staple **215** anchored in the vertebral body **159.**
Figure 11 depicts a coronal view of the clamped disc **100** during initial clamping. The sloped surfaces **170** of the compressors **111** rest on the surface of the annulus.
Figure 12 shows penetration of the sloped surfaces **170** with time. Further penetration is halted by the stops **173** resting on the sides of the vertebral body **159.**
Figure 13 depicts a coronal view of two unsymmetrical compressors **111** installed on a scoliotic vertebral segment.
Figure 14 shows correction or straightening of the scoliotic vertebral segment with time, by selectively elevating, wedging or shimming the concave side of the vertebral segment.
Figure 15 depicts a disc clamp **198** with thick compressors **111** installed on a disc **100** displaying spinal stenosis. The size of the clamp/compressors **198/111** is enlarged disproportionately to the disc **100,** for clarification.
Figure 16 shows penetration of the sloped surfaces **170** and plateau surface **171** with time into the disc **100** to thicken the intervertebral disc **100.**
Figure 17 depicts a coronal view of compressors **111** initially installed around a disc **100** displaying spinal stenosis. Bone spurs have grown around the vertebral body **159.**
Figure 18 shows penetration and shimming of the compressors **111** with time into the disc **100** to elevate disc height. The penetration is halted when the stops **173** rest on the vertebral body **159.**
Figure 19 shows that the compressors **111** can be modular components individually fitted on a disc clamp **198.**
Figure 20 depicts the modular compressor **111** comprising an annulus contact surface **119,** sloped surface **170,** plateau surface **171,** stop **173** and pivotal peg **172** for inserting into the clamp.
Figure 21 indicates a modular compressor **111** including a casing **188** with anchoring screws **187** and the disc contact portion of the compressor **111.**
Figure 22 depicts a vertical cross-sectional view of a compressor **111** with two stops **173,** an outer surface **233,** upper and lower plateau surfaces **171,** sloped surfaces **170** and annular contact surface **119.**
Figure 23 shows a compressor **111** with no stop and a very round annular contact surface **119.**
Figure 24 depicts a compressor **111** with multiple slopes in the sloped surfaces **170.**
Figure 25 shows a compressor **111** with unsymmetrical sloped surfaces **170.**
Figure 26 depicts a compressor **111** with tissue ingrowth openings **160** on the plateau surfaces **171** to promote annular ingrowth and stability of the compressor **111.**
Figure 27 shows a compressor **111** with non-parallel plateau surfaces **171.**
Figure 28 indicates the clamp **198** width measurement and the reach-in distance to stabilize the fastened clamp **198.**
Figure 29 depicts a typical strain vs. stress profile of nickel-titanium (nitinol) alloy suitable for fabricating into a disc clamp **198.**
Figure 30 indicates a compressor **111** pivotally fastened with a screw **187** to a bracket **139.**
Figure 31 shows a one-piece compressor **111** with a bracket **139.**
Figure 32 depicts the one-piece compressor **111** and bracket **139** fastened by two bolts **161** or screws onto the side of the vertebral body **159,** compressing the disc **100.**
Figure 33 shows a coronal view of disc **100** compression by the compressors **111** on brackets **139** fastened with bolts **161** and nuts **162** through the vertebral body **159.**
Figure 34 depicts a bolt **161** with two longitudinal slits **106** cut in series. The bolt **161** is made with elastic material, such as nickel-titanium (nitinol).
Figure 35 depicts the slits **106** being shimmed open and shaped, forming four elastic and compressible struts **107.** The length of the bolt **161** is elastically and resiliently shortened.
Figure 36 shows a sleeve **104** with a lumen **225** and four windows **114,** sized and configured to allow protrusion of the elastic struts **107** of the bolt **161,** as shown in Figure 35.
Figure 37 indicates the insertion of the bolt **161** with the elastic struts **107** being resiliently compressed and fitted within the sleeve **104** in an out-of-phase position.
Figure 38 depicts protrusion of the opened struts **107** from the windows **114** by turning the bolt **161** relative to the sleeve **104** from the out-of-phase to an in-phase position.
Figure 39 indicates a coronal view of a spinal stenosis segment fastened with two compressors/brackets **111/139** by two elastic bolts **161** containing slits **106** in out-of-phase position.
Figure 40 indicates disc **100** compression and penetration with time by the compressors **111,** activated or initiated by turning the elastic bolts **161** to in-phase position with the sleeve **104.**
Figure 41 depicts a compressor/bracket **111/139** installed on the concave curvature of a scoliotic vertebral segment.
Figure 42 shows disc **100** compression and penetration with time by the compressor **111** to correct or straighten the scoliotic vertebral segment.
Figure 43 indicates a biodegradable sleeve **218** restricting the elastic struts **107** of the bolt **161** from opening and elastically shortening.
Figure 44 shows a coil spring **125.**
Figure 45 depicts a coronal view of disc **100** compression by the compressor **111** and coil spring **125** assembly.
Figure 46 indicates shimming of the compressor **111** into the disc **100** with time, compressed by the coil spring **125.**
Figure 47 shows a spring **124** including of two connecting lift springs **121,** which can provide disc compression similar to the coil spring **125.**
Figure 48 indicates a compressor **111** with an elastic fastening means **127** installed at the anterior portion of a kyphosis vertebral segment.
Figure 49 shows correction of the kyphosis vertebral segment by disc **100** elevation and penetration of the compressor **111.**
Figure 50 depicts a disc compressor **111** on a lengthened bracket **139** designed to fuse the vertebral segment and elevate disc space.
Figure 51 shows spinal fusion and disc **100** compression with the lengthened compressor/bracket **111/139** fastened with bolts **161** or screws concealed in the indentation **164.**
Figure 52 indicates a coronal view of spinal fusion and disc **100** compression with the lengthened compressor/brackets **111/139** fastened on the vertebral bodies **139.**
Figure 53 indicates a coronal view of normal bulging of annular layers during axial compression.
Figure 54 shows annular delamination due to inward and outward bulging caused by aging or a dehydrated nucleus pulposus **128.**
Figure 55 depicts seepage of nucleus pulposus **128** through damaged annular layers, possibly from the weakened, delaminated annular layers.
Figure 56 indicates disc **100** compression by the compressors **111,** promoting inward annular bulging to minimize further delamination.
Figure 57 shows the sinuvertebral nerve **216** ingrowth into the disc **100,** causing discogenic pain.
Figure 58 depicts compression of the sinuvertebral nerves **216** by the compressors **111** to atrophy the nerves **216.**
Figure 59 depicts the insertion of a trocar **103** laterally through the bulging disc **100,** with the aid of a guide **185** (optional).
Figure 60 indicates the insertion of a dilator **230** over the trocar **103.**
Figure 61 shows the withdrawal of the trocar with the dilator **230** remaining in the disc **100.**
Figure 62 depicts the insertion of a bolt **161,** compressor **111** and washer **163** assembly into the dilator **230.**
Figure 63 indicates the withdrawal of the dilator to expose the thread **109** of the bolt **161.**
Figure 64 shows the installation of another compressor **111** onto the bolt **161** with washer **163** and nut **162.**
Figure 65 depicts disc **100** compression by tightening the nut **162** on the bolt **161.**
Figure 66 depicts fastening of a compressor/bracket **111/139** with a screw **187** through part of the disc **100** into vertebral body **159,** another screw **187** through the bracket **139** into the side of vertebral body **159.**
Figure 67 shows surgically inflicted bleeding sites **224** by a trocar **103** at the end plate **105** for annular adhesion and/or regeneration and a deep puncture for screw entry **217.**
Figure 68 depicts surgically inflicted bleed sites **224** at the end plate **105** by a curved trocar **103.**
Figure 69 shows a screw **187** through a compressor **111** with a trough **223** or indentation to conceal a screw head **226.**
Figure 70 shows the installation of the compressor **111** into the end plate **105** through a protruded disc. **100** impinging **184** on a nerve **102.**
Figure 71 shows disc **100** fastening by the compressor **111** to alleviate the impingement of an adjacent nerve **102.**
Figure 72 depicts a coronal view of the compressor **111** fastened through the outer portion of the disc **100** into the end plate **105** with bleeding sites **224** created to promote annular adhesion and regeneration.
Figure 73 depicts nerve impingement **184** from spondylolisthesis.
Figure 74 shows surgically inflicted bleeding sites **224** at the end-plate **105** by a trocar **103** to promote adhesion and reattachment between the disc **100** and vertebral body **159.**
Figure 75 depicts a rigid sleeve **220** sliding on an elastically curved trocar **103** with a label **221** on the handle indicating the direction of the curvature.
Figure 76 shows that the curvature of the elastic trocar **103** is resiliently straightened within the lumen of the sleeve **220.**
Figure 77 demonstrates that the end plate **105** can be reached even when the sleeve **220** is introduced perpendicularly to the disc **100.**
Figure 78 depicts a bulging disc **100** sandwiched between two vertebral bodies **159.** The bulges may result in spinal stenosis and/or segmental instability.
Figure 79 depicts disc **100** compression, stabilization and elevation with two compressors **111** anchored through the end plate **105** into the vertebral body **159.**
Figure 80 shows disc **100** thickening with the fastened compressor **111** to reduce spinal stenosis.
Figure 81 depicts disc **100** fastening with screws **187** anchoring into vertebral bodies **159,** above and below the intervertebral disc **100.**
Figure 82 shows a bolt **161** traversing through the end-plate **105** and the vertebral body **159** to fasten the compressor **111** with a nut **162** supported by a washer **163.**
Figure 83 depicts a compressor **111** with multiple tissue ingrowth openings **160.**
Figure 84 depicts a compressor **111** with outwardly curved tips **130** and tissue ingrowth openings **160** penetrating through the thickness of the compressor **111.**
**Figure 85** shows a resilient compressor **111** in an open or predisposed position.
Figure 86 depicts the resilient compressor **111** being constricted or folded within a delivery capsule **131.**
Figure 87 indicates the insertion of the delivery capsule **131** onto a protruded disc **100.**
Figure 88 shows the advancing screw **187** anchoring in the vertebral body **159** and expelling the compressor **111** from the capsule **131** onto the protruded disc **100.**
Figure 89 indicates disc **100** fastening with the compressor **111** in an expanded or compressed position.
Figure 90 depicts a stabilizer **134** inserted within the delivering capsule **131** to minimize tilting of the screw head **226** during disc **100** fastening.
Figure 91 shows a clamp/compressors **198/111** with large tissue ingrowth openings **160.**
Figure 92 shows bone ingrowth from upper and lower vertebral bodies **159** into the tissue ingrowth openings **160** of the clamp/compressors **198/111** leading to spinal fusion **234.**

### DETAILED DESCRIPTION OF THE EMBODIMENTS

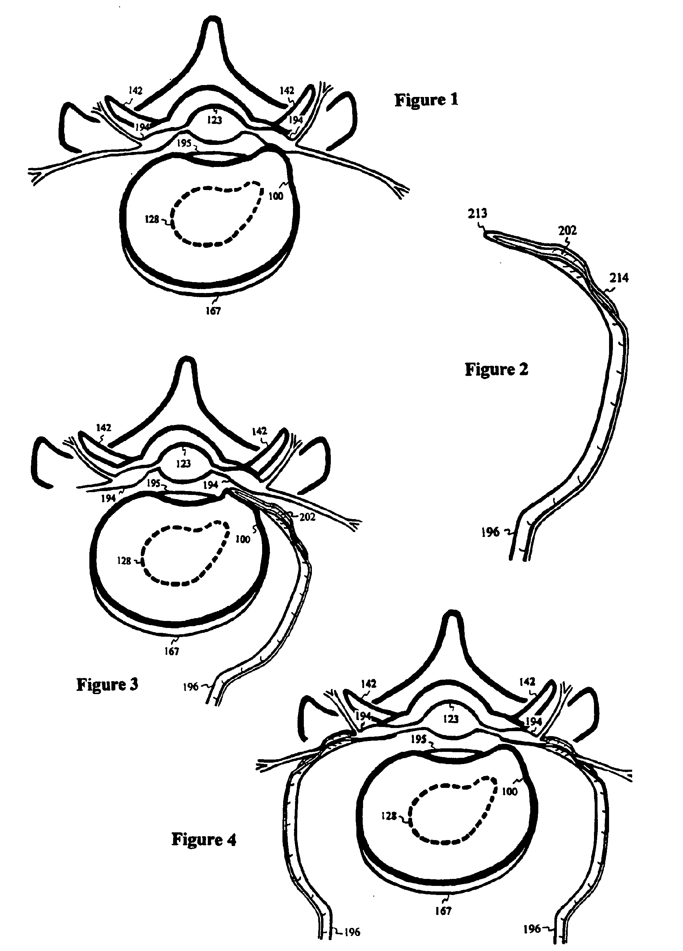

Figure 1 depicts a common nerve **194** impingement from a protruded disc **100** at or near the narrow channel of the neuroforamen. For protection during disc **100** repair, a nerve shield **196** contains a thin and blunt distal tip **213** for reaching into or near the neuroforamen, a trough **202** to partially surround and protect the nerve **194** and an open channel **214** for the nerve **194** to exit from the trough **202.** Through anterior or lateral incision, the nerve shield **196** is introduced by sliding over the bulging annulus of the disc **100,** as shown in Figure 3, to minimize potential damage to the ventral/dorsal ramus nerve root **194.** The shield **196** is then gently pressed against the partially surrounded nerve **194.** Similarly, another nerve shield **196** is used contralaterany to protect both nerves **194** existing from the neuroforamen, as shown in Figure 4.

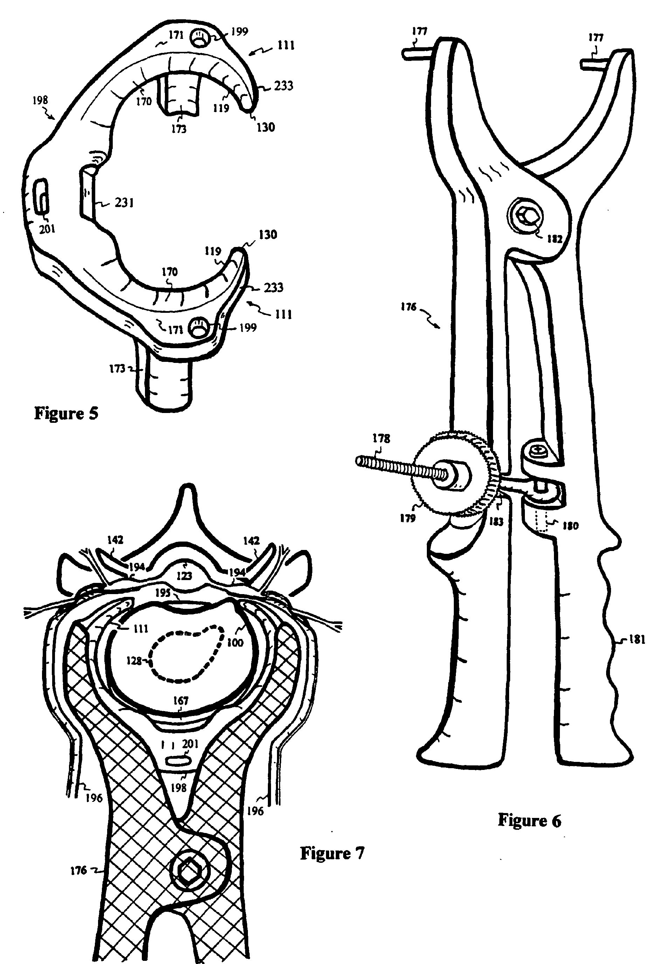

Figure 5 shows an elastic intervertebral disc clamp **198** with an annular contact surface **119,** a sloped surface **170,** a plateau surface **171** and stops **173** on the compressors **111** portions. The saddle-shaped compressors **111** are used to bracket the dysfunctional disc **100** bilaterally. The clamp/compressor **198/111** has a support mount **201,** an indentation **231** and two widening mounts **199** for engagement with a widening tool, as shown in Figure 6. The clamp **198** can be made with nickel-titanium, nitinol, or other elastic alloy or polymers. Figure 6 shows a clamp-widening tool **176** equipped with clamp grabbers **177** for engaging with the widening mount **199** on the compressors **111,** a pivotal joint **182,** handles **181** and a locking mechanism capable of slowly releasing the compressor **111.** The mechanism contains a hinge **180** anchoring a lock screw **178** fastened with a lock wheel **179.** The lock screw **178** is sized and configured to fit into a lock slot **183** to lock the handle **181** of the widening tool **176.** For quick release of the handle **181,** the lock screw **178** can be picked up from the slot **183.** For slow release, the lock wheel **179** can be rotated to slowly open the handle **181,** thus slowly closing the disc clamp **198.**

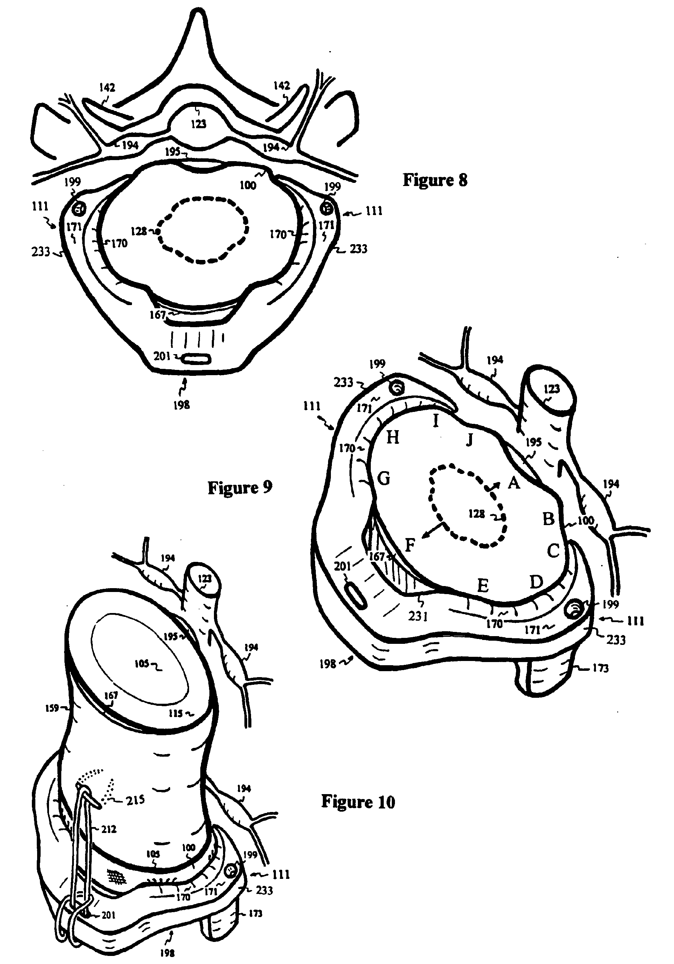

Figure 7 depicts widening and placement of the disc clamp **198** by the widening tool **176.** The clamp **198** fits around the intervertebral disc **100,** while nerves **194** are protected by nerve shields **196.** The distal tips of the compressors **111** are thin and tapered to prevent impingement of the nerve **194.** The clamp **198** is then slowly released by dialing the lock wheel **179,** as shown in Figure 6. Figure 8 shows the disc **100** being clamped by the disc clamp **198** as the compressors **111** press the bulging annulus inwardly to alleviate nerve **194** impingement. The size of the clamp/compressor **198/111** is enlarged disproportionately to the disc **100,** for clarification. Figure 9 indicates the locations of compression from the disc clamp **198.** The preferred compressions are at areas C and I, common protruding locations of the disc **100,** with areas E and G as supporting locations. From a disc **100** fastening cadaveric study, nearly the entire disc **100** was distracted, elevated and slightly lengthened from compression by the compressors **111.** The portion of annulus remote to the compressors **111** was also distracted, pulling inward. The previously protruded areas B and J in Figure 9 would similarly be distracted as well. Annulus distraction is wide spread and far reaching, way beyond the area of direct compression. The benefit of the far-reaching capability of the compressors **111** is most significant in repairing annular impingements commonly occurring around the narrowed neuroforamen. The compressors **111** can be fastened a distance away from the impinging neuroforamen, yet the distraction of the annulus can draw in the distant bulge, alleviating the impingement. Alternatively, decompressing the nerve impingement within the neuroforamenal region (the hidden zone) surrounded by the disc **100,** vertebral body **159,** pedicle and facet joint **129** is very invasive using current surgical procedures, and it may result in increased scarring and a permanently weakened spine.

As the disc **100** is compressed by the body weight, area F located at the indentation **231** and area A are allowed to naturally and resiliently bulge as indicated by arrows in Figure 9, since they are least restricted by the clamp **198.** The thinning or tapering of the distal tips of the compressors **111** are essential to avoid nerve **194** impingement, as shown in Figures 8 and 9. To minimize possible damage to the disc **100,** the annular contact surfaces **119** of the compressors **111** are generally cylindrical or blunt, thickening into the sloped surface **170,** as shown in Figure 5, with an optional plateau surface **171.**

To prevent migration of the clamp **198,** especially during initial installation, an elastic strap **212** is threaded through the support mount **201** and secured by a staple **215** anchored in the vertebral body **159,** as shown in Figure 10. More than one strap **212** and staple **215** can be used. The strap **212** can be a biodegradable suture or material to initially secure the clamp **198** until the sloped surfaces **170** of the compressors **111** penetrate the annulus and adequately secure the clamp/compressors **198/111.**

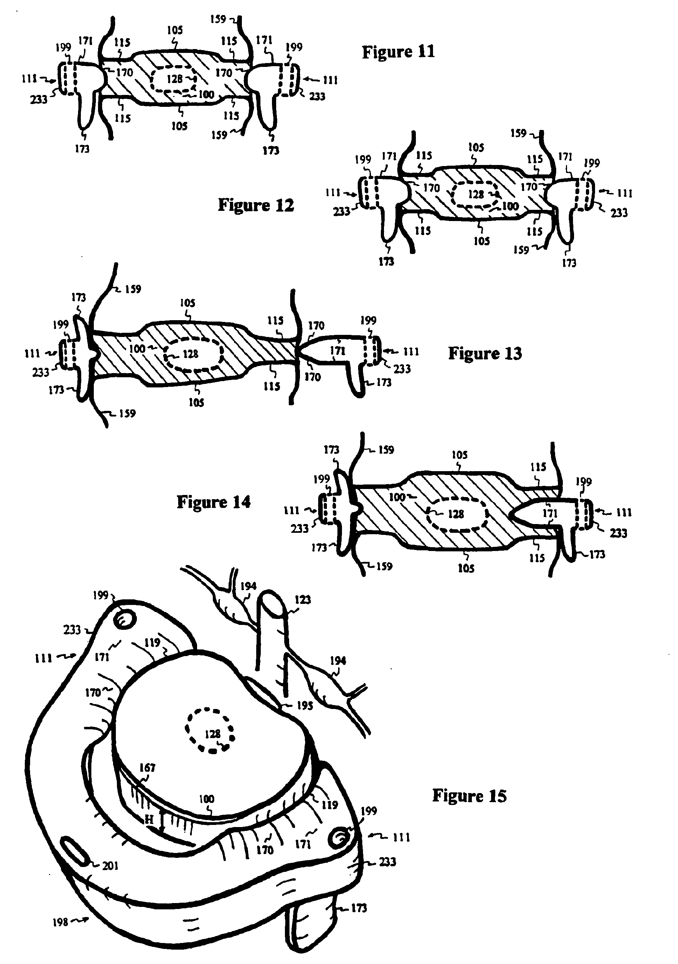

Figure 11 depicts a coronal view of initial clamping of the disc **100** with the sloped surface **170** resting on the disc **100.** With time, the sloped surface **170** of the compressor **111** slowly penetrates into the disc **100** until the stops **173** gently rest on the lateral side of the vertebral body **159** below the disc **100,** as shown in Figure 12. The stop **173** is a protrusion, a small wall or a leg from the under side of the compressor **111.** The clamp/compressors **198/111** is designed to compress the protruded annulus, alleviating the nerve impingement. The clamp/compressors **198/111** also restricts, support and stabilize the bulging annulus to alleviate pain from segmental instability.

Current surgical treatment for scoliosis is invasive, most frequently done on young female patients to correct the deformity. Instrumentation failure or breakage of pedicle screws is likely after decades of wear and tear, mandating a second surgery. Figure 13 depicts a coronal view of a scoliotic vertebral segment initially clamped and compressed by the unsymmetrical compressors **111** of a disc clamp **198** (not shown). The concave side of the curved vertebral segment is fitted with a thick compressor **111** comprising a wide plateau surface **171,** while the convex side of the vertebral segment is fitted with a thin compressor **111** containing a narrow or absent plateau surface **171.** Figure 14 shows correction or straightening of the scoliotic vertebral segment with time, by selectively wedging, shimming and elevating the concave side of the curved vertebral segment and by inserting the plateau surface **171** of the compressor **111** between the dense epiphyses **115.** To straighten the entire spine, multiple selective disc **100** elevations are required, much as multiple pedicle screws and instrumentation are used in current procedures. Scoliosis is corrected through selective shimming by the compressor **111** to alter the lateral curvature of the spine. Nickel-titanium compressors **111** are expected to be durable between the epiphyses **115;** and the clamp **198** is under minimal strain after settlement in the disc **100.** Thus the clamp/compressors **198/111** are expected to be long lasting, perhaps even permanent without revisional surgery.

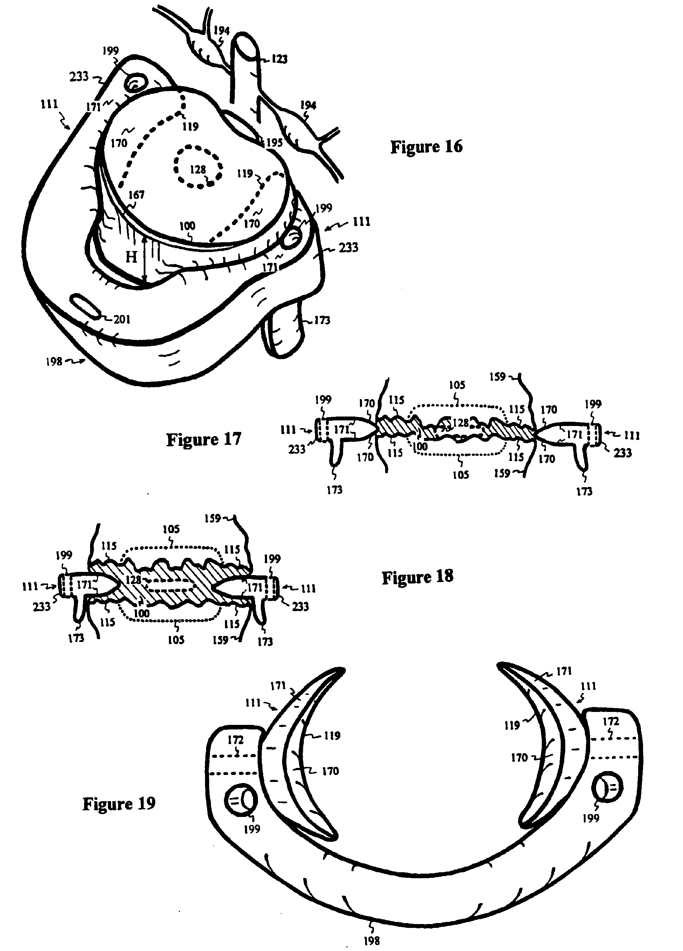

Spinal stenosis is a progressive disorder. Figure 15 depicts a flattened disc **100** with a dehydrated nucleus pulposus **128.** The initial disc height, H, is indicated at the anterior portion of the disc **100.** A clamp **100** with two symmetrical compressors **111** with wide plateau surfaces **171** is clamped around the flattened disc **100.** The size of the clamp/compressors **198/111** is enlarged disproportionately to the disc **100,** for clarification. Gentle compression and wedging action of the clamp/compressors **198/111** allow time for the annulus to grow and thicken. The surrounding ligaments, including the posterior **195** and anterior **167** longitudinal ligaments and facet joint ligaments, also require time to lengthen. As the sloped surface **170** wedges into the disc **100,** the plateau surfaces **171** establish stable positions between epiphyses **115** to thicken the disc **100** and provide elevated disc height, H, as shown in Figure 16. With elevated intervertebral disc space, nerve impingement caused by spinal stenosis is minimized or alleviated. Disc **100** penetration by the compressors **111** halts when the stops **173** reach the lateral surfaces of the vertebral body **159,** in this case below the disc **100.** Figure 17 depicts a coronal view of a clamp **198** (not shown) and compressors **111** initially clamped around a disc **100** sandwiched by bone spurs, common among patients with spinal stenosis. With time, Figure 18 shows wedging and penetration of the sloped surfaces **170** followed by the plateau surfaces **171** into the disc **100** between the epiphyses **115** of the vertebral bodies **159.** Thus, disc **100** height increases to alleviate nerve impingement common among spinal stenosis patients. Penetration of the compressors **111** halts when the stops **173** rest upon the vertebral body **159** below the disc **100.** The plateau surface **171** maintains disc height without the need of further compression. In contrast to current surgical techniques, which cut or bur away anatomical structure to make room for the progressively narrowing disc space, the clamp/compressors **198/111** restore or increase the disc **100** height to minimize or alleviate nerve impingement.

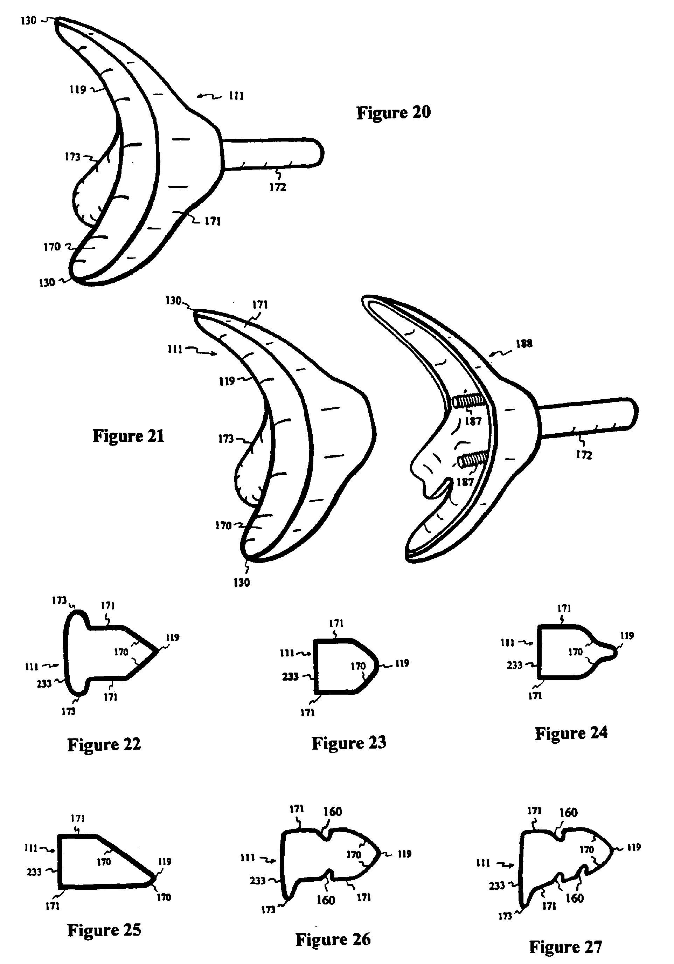

The clamp **198** and the compressors **111** can be made separately as modular components assembled into a device as shown in Figure 19. The vertical cross-section of the clamp **198** can be semi-circular, elliptical, circular or another shape with blunt surfaces to prevent abrasion to the disc **100,** abdominal contents or blood vessels. The saddle-shaped compressor **111** contains a pivotal peg **172** for inserting into the clamp **198,** a smooth and blunt annular contact surface **119,** a sloped surface **170,** a plateau surface **171** and a stop **173,** as shown in Figure 20. The concave curvature of the annular contact surface **119** of the compressor **111** is designed to conform and fit partially around the disc **100.** Since most discs **100** are not circular, the concave or crescent curvature of the annular contact surface **119** is likely to be complex or to contain multiple radiuses in order to conform to the surface of a disc **100.** One of the tips **130** of the compressor **111** is particularly thin and tapered, designed to minimize nerve impingement especially near the neuroforamen. The compressor **111** can also be made with modular components, as shown in Figure 21. The annular contacting part of the compressor **111** can be made with biocompatible polymer, such as polyurethane, polypropylene, polyethylene, PEEK, Delrin, polysulfone, polytetrafluoroethylene, polycarbonate, ultra high molecular weight polyethylene or other low friction polymer. The casing **188** with pivotal peg **172,** as shown in Figure 21, can be made with stainless steel, titanium, nickel-titanium or metal, or even a polymer. The components can be assembled with screws **187** also shown in Figure 21.

The thickness, curvature, surfaces **119, 170, 171** and/or stops **173** of the compressor **111** can vary to accommodate proper disc **100** compression. Figure 22 depicts a vertical cross-sectional view of a compressor **111** containing two stops **173** to improve stability. Figure 23 shows a compressor **111** with no stop **173** and a round annular contact surface **119** for gentle compression. Figure 24 indicates a compressor **111** with multiple sloped surfaces **170** to gain rapid annular penetration and provide initial stabilization of the clamp **198.** Figure 25 shows an unsymmetrical slope **170** for shimming into a disc **100** to correct or straighten some kyphosis, scoliosis, lordosis or other spinal deformity. Figures 26 shows tissue ingrowth openings **160,** indentations or troughs to promote annular ingrowth and stabilization of the compressor **111.** The plateau surfaces **171** with tissue ingrowth openings **160** can also be non-parallel to each other, as shown in Figure 27, to correct and stabilize some spinal deformities.

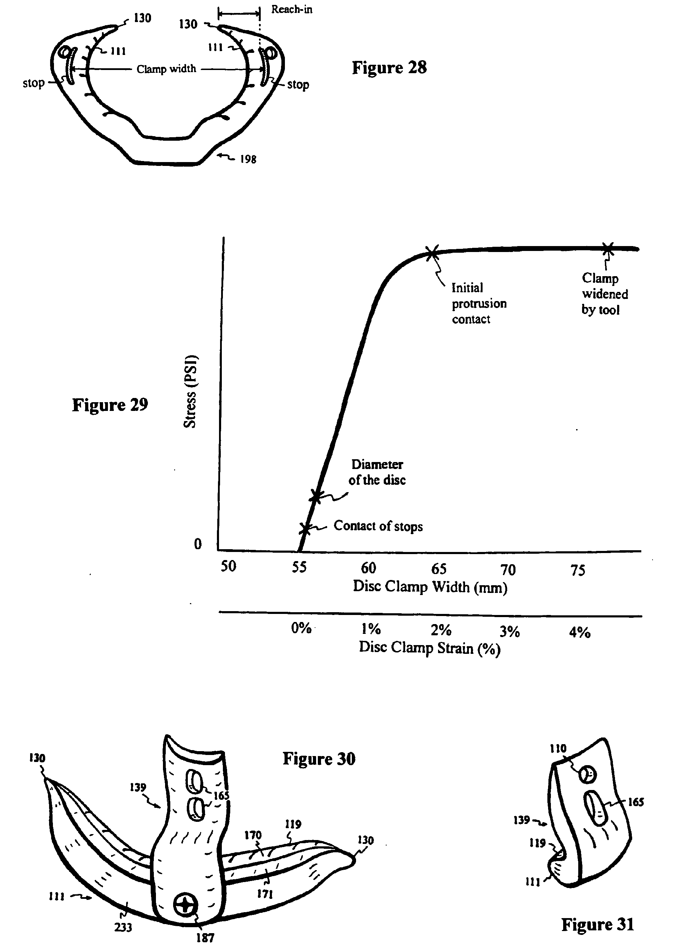

For compressive strength, biocompatibility and durability, nickel-titanium perhaps is the most suitable material for fabricating the clamp **198.** The clamp width and reach-in portions are defined in Figure 28. The reach-in portions of the clamp **198** are essential for securing the initial fastening and clamping of the disc **100.** The distal tips **130** are tapered to prevent nerve impingement by the reach-in portions of the clamp **198.** Figure 29 is a typical strain vs. stress profile of nickel-titanium alloy, a super elastic alloy suitable for fabricating into a disc clamp **198.** Various compressive stages of a nickel-titanium clamp **198** are also indicated in Figure 29. The compressive force is greatest initially when it presses in the annular protrusion. As the protrusion is compressed, it relieves the strain of the clamp **198;** the compressive force of the clamp **198** rapidly weakens. When the stops **173** reach the vertebral body **159,** the compressive force is insignificant, minimizing erosion on bone and annulus. Since the stress on the clamp **198** is minimal after protrusion compression, continual erosion of the disc **100** may not occur even in the absence of the stops **173** on the compressors **111.**

The clamp/compressors **198/111** can also be installed through a lateral incision. A widening tool is modified to hold the clamp/compressors **198/111** laterally. The modified tool is also used as an extension to install the device **198/111** in the patient. Lateral insertion and device **198/111** maneuvering can minimize possible damages from excessive tissue retraction, especially for intervertebral discs **100** surrounded by blood vessels, muscles and nerves. For example, the L3-4 disc **100** is sandwiched bilaterally by the Psoas major muscles containing lumbosacral nerve roots, sensitive to excessive retraction. Aorta and inferior vena cava are anterior to the disc **100.** To compress the L3-4 disc **100,** the open side of the widened C-like clamp/compressors **198/111** is oriented vertically either superiorly or inferiorly to the patient, to make the insertion as thin as possible. Through a lateral incision, the widened and vertically oriented C-like clamp/compressors **198/111** is inserted between the L3-4 disc **100** and the blood vessels (aorta and inferior vena cava) anterior to the disc **100.** The clamp/compressors **198/111** is then slowly rotated to orient the open side posteriorly, placing both compressors **111** laterally around the L3-**4 100.** The clamp/compressors **198/111** is then slowly released to compress the disc **100,** followed by retrieval of the widening tool.

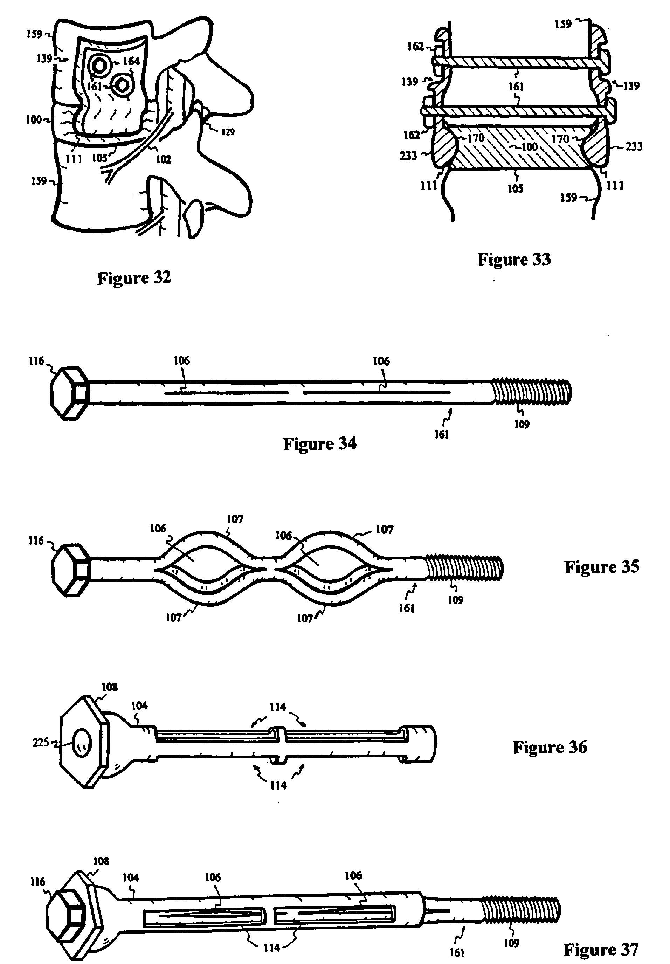

The compressor **111** can also be fastened to a bracket **139** by a screw **187,** as shown in Figure 30. The bracket **139** is equipped with slits **165** for bolts or screws to fasten into the vertebral body **159,** thus compressing the protruded disc **100** with the compressor **111.** The compressor **111** can also be made with the bracket **139** in one-piece as shown in Figure 31. Figure 32 depicts compression of the protruded disc **100** by the compressor/bracket **111/139** fastened by bolts **161** or screws into the vertebral body **159** with the heads of the bolts concealed in the indentation **164** of the bracket **139.** Figure 33 shows a coronal view of bilateral disc **100** compression fastened with compressor/bracket **111/139** and bolts **161** through the vertebral body **159.** In essence, the brackets **139** serve similar function as the stops **173** with attachment holes **165, 110.**

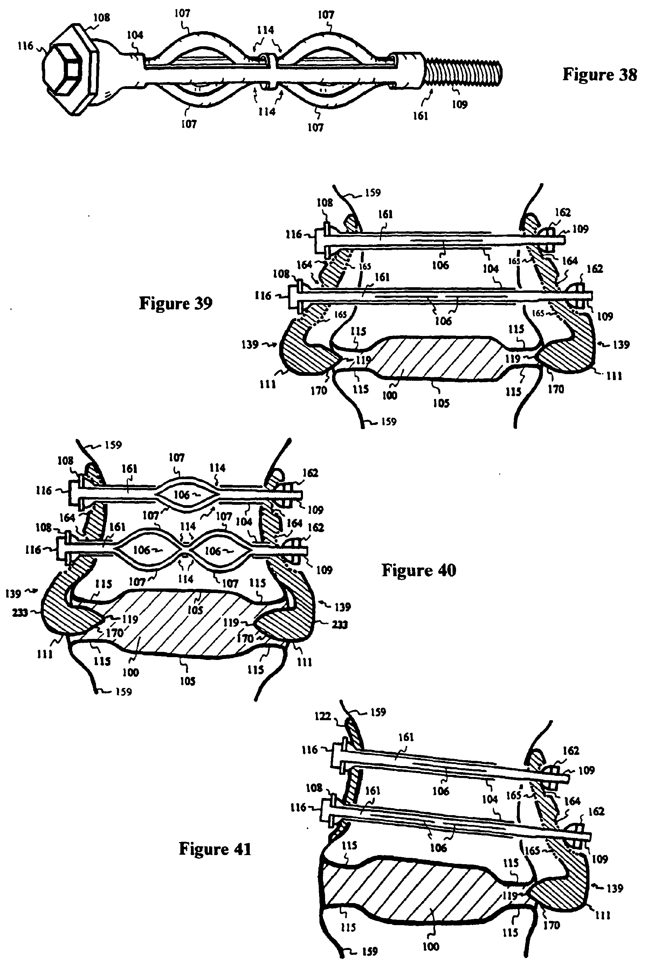

**Figure 34** depicts a bolt **161** with two longitudinal slits **106** cut along the length of the bolt **161.** The bolt **161** is made with elastic metal, such as nickel-titanium. The slits **106** can be cut with laser, water jet, wire or sinker EDM (electron discharging machine). Figure 35 depicts the slits **106** after being shimmed open and shaped to form four elastic and compressible struts **107.** For nickel-titanium bolts **161,** the struts **107** are shaped by inserting shims or fixtures, heating the shimmed bolts **161** to about 500°C for 5-10 minutes, then quickly quenching the heat-treated bolt **161** in cold water before removing the fixtures. It is also possible to mold or cast a bolt **161** with elastic and compressible struts **107** already in open positions, as shown in Figure 35. Elastic polymers can also be used to mold into an elastic bolt **161** with compressible struts **107.** With the struts **107** open, the length of the bolt **161** is elastically or resiliently shortened. Figure 36 shows a sleeve **104** with lumen **225** and four windows **114** sized and configured for the protrusion of the elastic struts **107** of the bolt **161.** Figure 37 indicates the insertion of the bolt **161** with the elastic struts **107** being resiliently compressed and fitted within the sleeve **104.** The struts **107** and the windows **114** are in an out-of-phase position, where the windows **114** and direction of struts **107** deployments do not overlap. The length of the bolt **161** in out-of-phase position within the sleeve **104** is longer than the length of the bolt **161** with open struts **107,** as shown in Figure 35. Figure 38 depicts turning of the bolt **161** relative to the sleeve **104** or turning of the sleeve **104** relative to the bolt **161,** from the out-of-phase position to an in-phase position, where the windows **114** align with the directions of struts **107** for deployment. As a result, the elastic struts **107** protrude out of the windows **114** and the overall length of the bolt **161** is elastically or resiliently shortened.

Figure 39 shows a coronal view of a vertebral motion segment with decreased disc height or symptoms of spinal stenosis. Two disc-compressor/brackets **111/139** are laterally anchored with two elastic bolts **161** containing slits **106** within two sleeves **104** in out-of-phase positions. The round sleeve head **108** and round nut **162** are designed to allow pivotal movement of the compressor/brackets **111/139** during disc **100** compression. The deployment of the struts **107** is activated or initiated by rotating the sleeves **104** from out-of-phase to in-phase positions, allowing the struts **107** to protrude out of the windows **114** of the sleeves **104** and to provide elastic or resilient inward pulling tension on both compressors/brackets **111/139.** Similar to the clamp/compressor **198/111,** the elastic disc **100** compression allows time for the surrounding ligaments to slowly extend and the annulus of the disc **100** to gradually thicken. As a result, tissue damage is minimized and disc **100** height is elevated to alleviate spinal stenosis, as indicated in Figure 40. For ease of illustration, Figure 40 shows that the plane of the deployed struts **107** is perpendicular to the end plate **105,** but ideally the plane of the deployed struts **107** should be parallel to the end plate **105** to maximize the spread of the struts **107** without interfering with the end-plate **105.** Therefore, a marking on the bolt head **116** visible to the surgeon can be helpful to identify the plane of struts **107** deployment.

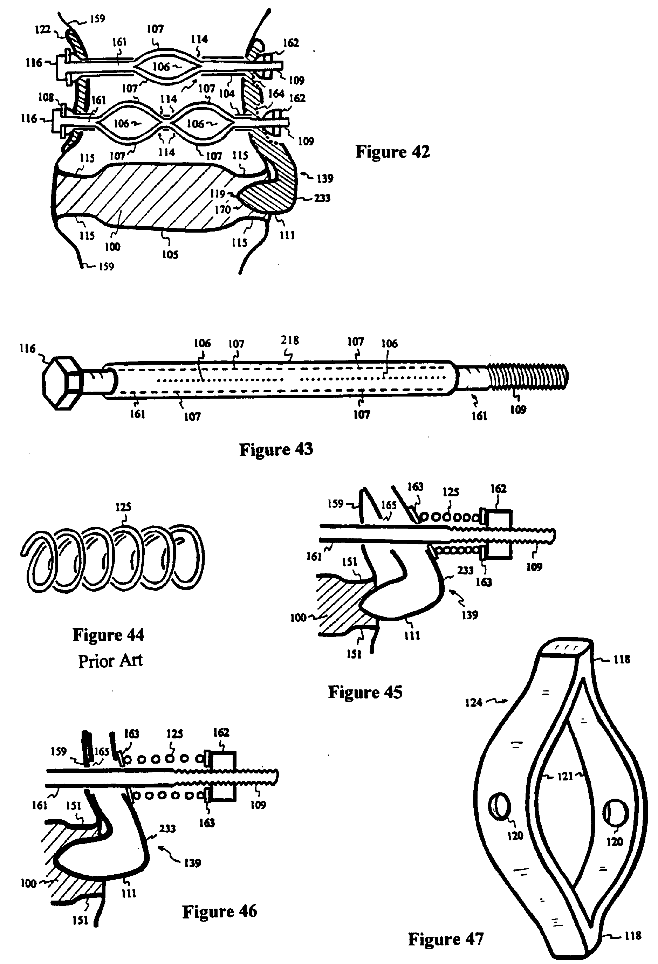

Figure 41 depicts a mono-lateral disc **100** compression into the concave side of the curved scoliotic vertebral segment. Figure 42 shows activation of elastic fastening by setting the bolt **161** and sleeve **104** to the in-phase position, slowly wedging the compressor **111** into the concave side of the curved spine to correct or straighten the scoliotic vertebral segment. To correct the entire scoliotic spine, multiple shimmings can be done in multiple scoliotic segments. The degree of individual shimming can be individually selected or fitted with different thicknesses and shapes of the compressor **111.** The plateau surfaces **171** of the compressor **111** can be non-parallel, as shown in Figure 27, to optimize the fit and correction. The plateau surfaces **171** can also be indented with a tissue ingrowth opening **160,** also indicated in Figure 27, to promote annular ingrowth and minimize outward slippage of compressor **111.**

Figure 43 indicates a degradable sleeve **218** holding or restricting the elastic struts **107** of the bolt **161** from opening. The rate of strut **107** opening is determined by the rate of degradation of the degradable sleeve **218.** The major benefit to the degradable sleeve **218** is the elimination of the step of turning from the out-of-phase to the in-phase position. Furthermore, gradual opening of the struts **107** may be preferred with a slowly eroding degradable polymer to gently and gradually compress and shim into the disc **100.** The degradable sleeve **218** can be made with polylactide, polyglycolide, poly(lactide-co-glycolide), polycaprolactone, polydioxanone, polyanhydride, trimethylene carbonate, poly-beta-hydroxybutyrate, polyhydroxyvalerate, poly-gama-ethyl-glutamate, poly(DTH iminocarbonate), poly(bisphenol A iminocarbonate), poly-ortho-ester, polycyanoacrylate and polyphosphazene. There are natural biodegradable materials, including collagen, gelatin, cellulose, chitin and dextran. Many of these biodegradable materials are not biocompatible in bone or in disc **100.** However, the elastic bolt **161** and the degradable sleeve **218** combination can be used in other industries to provide elastic tensile fastening. The degradation can be initiated by water. For implant use, polylactide, polyglycolide or poly(lactide-co-glycolide) is most promising for making the degradable sleeve **218.**

It is possible to have both elastic bolt **161** and sleeve **218** biodegradable for bone joining or tissue fastening. Degradation time for DL-polylactide is 12-16 months; 50/50 lactide and glycolide co-polymer is 1-2 months. The bolt **161** with open struts **107** can be made by injection molding with DL-polylactide (modulus 1.9 Gpa) and the sleeve **218** with 50/50 lactide and glycolide. Initiated by the degradation of the sleeve **218** within two months, the resilient strength of the bolt **161** begins. After 16 months, hopefully the wound has healed and the bolt **161** and nut **162** will also degrade.

Similar to the elastic bolt **161,** a coil spring **125** as shown in Figure 44 can also provide compression onto the compressor/bracket **111/139.** Figure 45 depicts a coronal view of disc **100** compression by a bolt **161,** compressor/bracket **111/139,** washer **163,** compressed coil spring **125,** another washer **163** and nut **162.** Figure 46 shows disc **100** compression and compressor **111** shimming activated by the coil spring **125.** Other type of springs can also be used. Figure **47** shows two connecting lift springs **121** curving or arching outwardly. The springs **121** are connected at both ends **118,** and a screw hole **120** lies near the center of both springs **121.** The lift springs **121** can be used as the coil spring **125** in Figures 45 and 46 to elastically compress the intervertebral disc **100.**

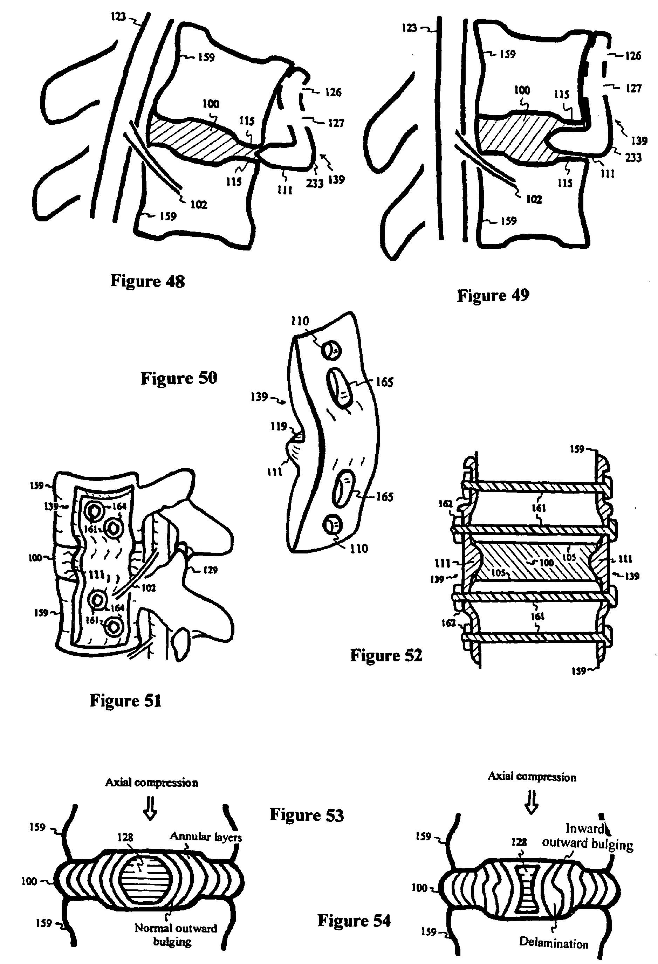

**Figure 48** indicates a compressor/bracket **111/139** installed anterior to a kyphotic vertebral segment. The bracket **139** is anchored by a pivoting means **126** and an elastic fastening means **127** onto the vertebral body **159.** With time, the compressor **111** shims into the disc **100** to correct and straighten the kyphotic bend as shown in Figure 49. The bracket **139** can also be made with elastic or resilient material installed under strain to compress into the disc **100.**

The compressor/bracket **111/139** can also be lengthened to serve dual functions: disc **100** compression and spinal fusion, as shown in Figure 50. Differing from the currently existing fusion plate, the extended compressor/bracket **111/139** compresses and thickens the disc **100** to increase disc space and possibly alleviate nerve impingement. The extended bracket **139** contains a compressor **111** near the mid-portion and screw/bolt holes **110** or slits **165** above and below the compressor **111.** Figure 51 depicts spinal fusion and disc compression with the extended compressor/bracket **111/139.** A coronal view of spinal fusion and disc compression with two compressors/brackets **111/139** fastened on the vertebral bodies **159** is shown in Figure 52. For the best results, the bolts **161** or screws are fitted in the slits **165** and evenly fastened to compress the disc **100** and distract the vertebral bodies **159.** Then holes are then created in the vertebral bodies to fit bolts **161** or screws through the bracket holes **110** and to further secure the bracket **139.** Disc **100** compression with spinal fusion is expected to provide disc height elevation, which may be particularly suitable for severe segmental instability or spinal stenosis. Using current technique, disc heights commonly decrease after intervertebral body fusion (Watkins R., et. al., Comparison of Disc Space Heights after Anterior Lumbar Interbody Fusion, Spine 14(8):876-878, 1989).

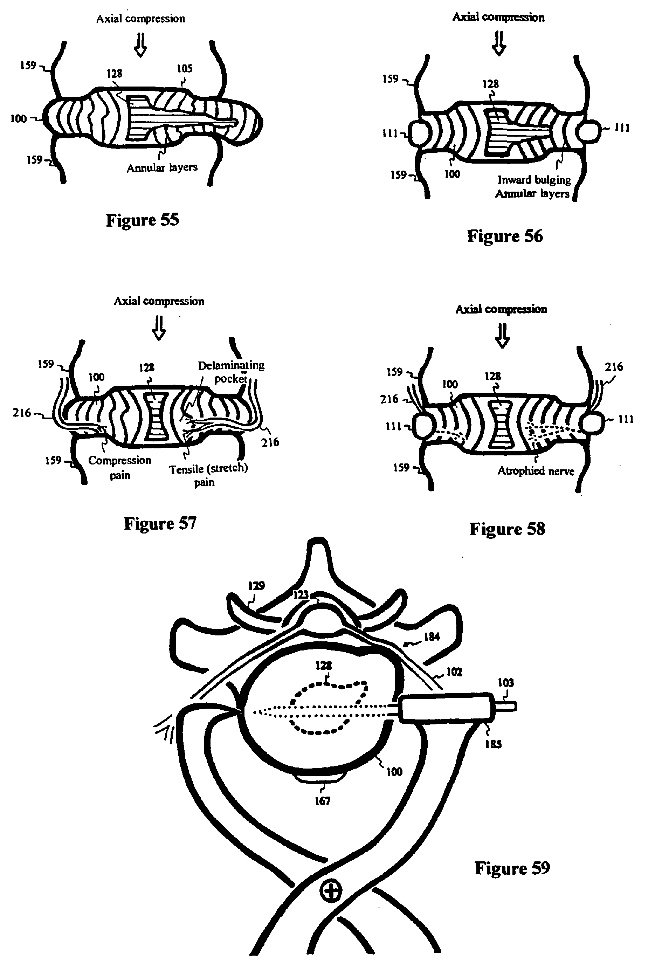

Figure 53 depicts a mid-coronal view of a vertebral segment with normal outward bulging of the annular layers during axial compression. As the nucleus pulposus **128** ages, dries out or degenerates, the annular layers exhibit both inward and outward bulging during similar axial compressions (Seroussi R.E. et. al., Internal Deformations of Intact and Denucleated Human Lumbar Discs Subjected to Compression, Flexion, and Extension Loads, Journal of Orthopaedic Research, 7:122-131, 1989; Meakin J.R., Replacing the nucleus pulposus of the intervertebral disc, Clinical Biomechanics 16:560-565, 2001). It is speculated that the inward-outward bulging causes delamination in the inner core of the annular layers, as shown in Figure 54. The delaminated annular layer is thin, unsupported and vulnerable to tearing. Usually, the delamination begins at the layers near the aging nucleus pulposus **128** and leads to seepage of nucleus pulposus **128** and disc **100** protrusion, as shown in Figure 55, (Goel V.K. et. al., Interlaminar Shear Stresses and Laminae Separation in a Disc, Spine, 20(6): 689-98, 1995). The compressors **111** provide inward compression to the disc **100,** flatten the protrusion and promote inward bulging to minimize the progression of annular delamination and to halt the deterioration of the defective disc **100,** as indicated in Figure 56. Disc **100** compression by the compressor **111** may also collapse and seal the seeping channels of nucleus pulposus **128** in a herniated disc **100** to minimize chemical irritation to nerves **102.**

Chronic low back pain is generally thought to be caused by nerve **102** impingement. However, MRI often fails to show impingement of neural structures, even in the presence of sciatica. Furthermore, saline injection, discography and compression of the longitudinal spinal ligaments can reproduce back pain and sciatica. These observations have led to re-examination of the pathways and distribution of nociceptive (pain sensing) nerve endings in healthy and diseased spines. In the healthy disc **100,** only the outer third of the annulus is innervated. But among patients with chronic low back pain, nerves extend into the inner third of the annulus, some even into the nucleus pulposus **128** (Freemont A.J. et. al., Nerve ingrowth into diseased intervertebral disc in chronic back pain, The Lancet, Vol. 350, July 19:178-181, 1997). Nerve ingrowth in connective tissue is normally a sign of repair in progress. However, similar to the articular cartilage in joints, the healing progress of annulus is very slow and poor. Figure 57 depicts the ingrowth of sinuvertebral nerves **216** conducting the sensation of tensile or stretching pain from the delaminated pockets within the degenerating disc **100.** Sinuvertebral nerves **216** normally grow from the surface into the annulus only when the disc **100** begins to degenerate. Figure 58 depicts compression of the sinuvertebral nerves **216** leading into the degenerative disc **100** by the compressors **111.** With prolonged and intense compression from the compressors **111,** the sinuvertebral nerves **216** are expected to cease transmitting signals of pain from the degenerative disc **100** and atrophy within days, thus alleviating pain without discectomy.

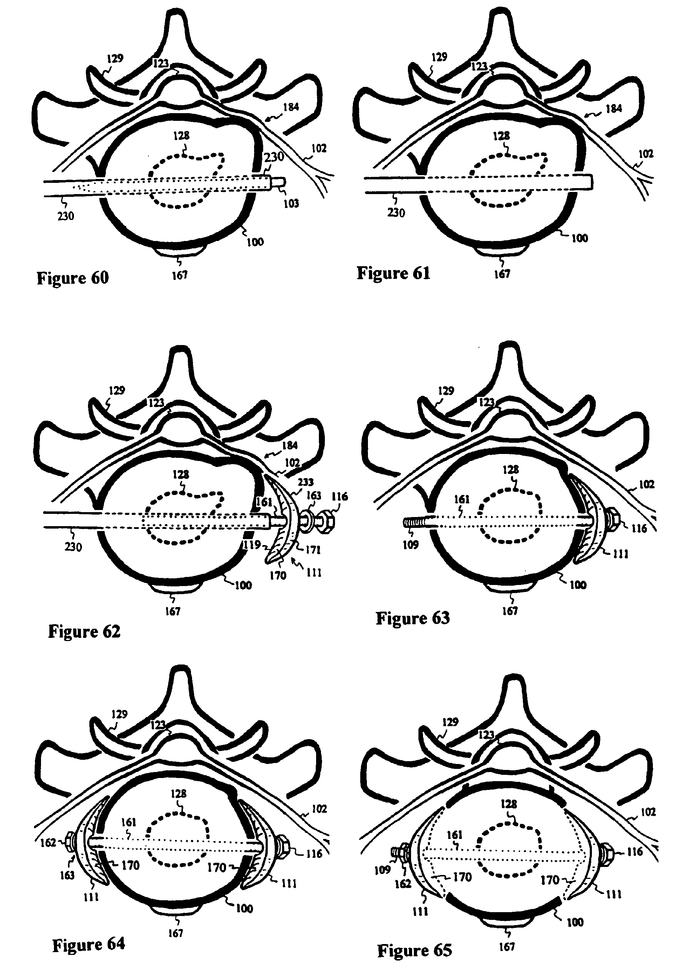

The compressors **111** can also be installed through a protruded disc **100.** With the aid of a trocar guide **185,** Figure 59 depicts the insertion of a trocar **103** laterally through the protruded disc **100** impinging **184** upon a nerve **102.** Insertion of the trocar **103** and compressors **111** can be done endoscopically through a lateral incision as well as through the anterior approach shown in Figure 59. Figure 60 indicates the insertion of a dilator **230** over the trocar **103.** Then the trocar **103** is withdrawn while the dilator **230** remains in the disc **100,** as shown in Figure 61. Figure 62 depicts the insertion of a bolt **161,** an arcuate compressor **111** and washer **163** assembly into the dilator **230.** Figure 63 indicates the withdrawal of the dilator **230** to exposure the thread **109** of the bolt **161.** Figure 64 shows the installation of another compressor **111** onto the bolt **161** with washer **163** and nut **162.** Figure 65 depicts tightening of the bolt **161,** nut **162,** compressors **111** and washer **163** assembly to fasten the bulging disc **100** with the sloped surface **170** embedding into the disc **100.** For elastic compression, the resilient bolt **161** with elastic struts **107** can be used with the sleeve **104,** as shown in Figure 37, or with the biodegradable sleeve **218** in Figure 43.

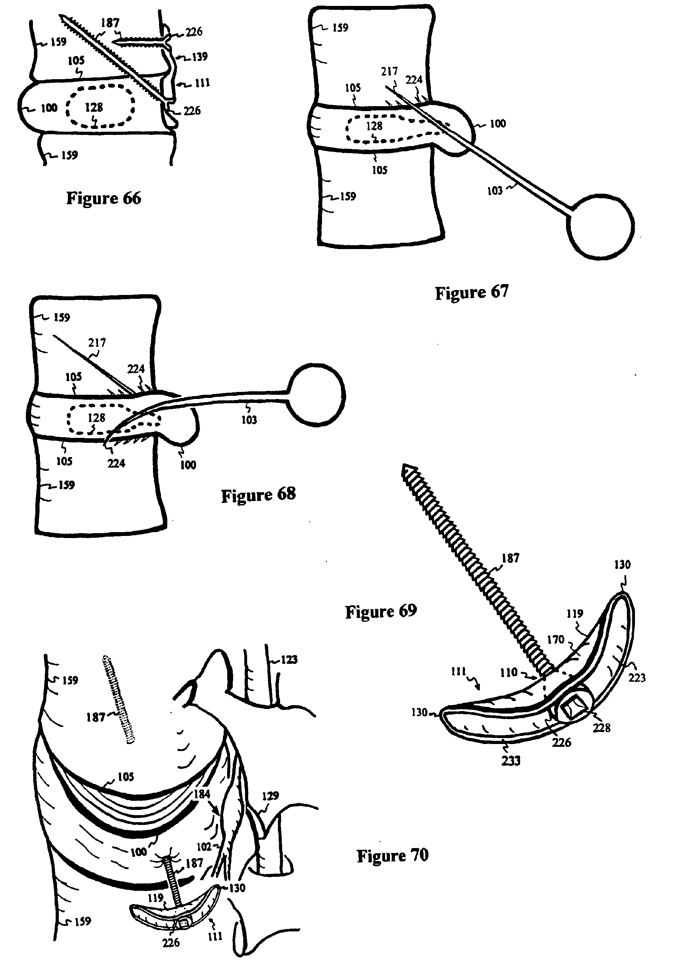

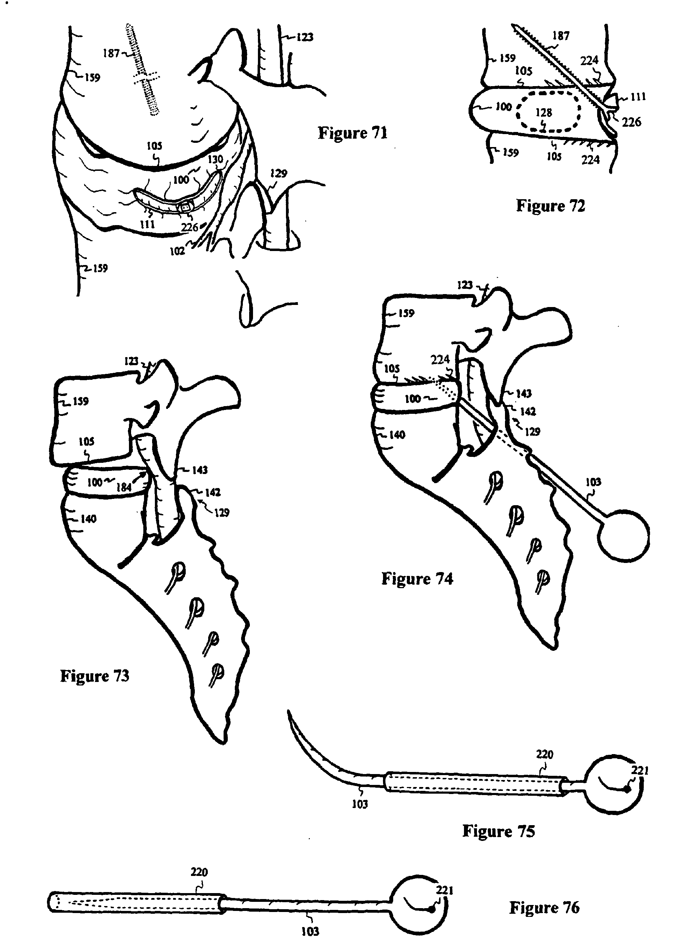

The compressor **111** can also be fastened through the outer layers of the disc **100,** and/or with a bracket **139** fastened on the vertebral body **159,** as shown in Figure 66. The screw entry **217** can be made with a trocar **103,** as shown in Figure 67. To enhance annular reattachment and/or regeneration of the otherwise slow healing, avascularized annulus, bleeding sites **224** at the end-plate **105** are created by the trocar **103** through the bulging disc **100,** as shown in Figure 67. The entry of the trocar **103** depicted in Figure 67 is slanted or angled upward, able to fit between the superior and inferior surfaces of the laminae, to prevent or minimize laminectomy. **Figure 68** shows a curved trocar **103** inflicting bleeding sites **224** in both superior and inferior end plates **105,** through a posterior/lateral approach. A saddle-shaped compressor **111** is shown in Figure 69 with a cylindrical annular contact surface **119,** sloped surface **170,** round contour tips **130,** a screw hole **110** and a trough **223** or indentation to conceal the screw head **226** of a screw **187.** Figure 70 depicts penetration of the screw **187** through the outer portion of a protruded disc **100** and the end plate **105** into the vertebral body **159.** Figure 71 shows compression of the protruded disc **100** by the compressor **111** fastened by the screw **187** anchored in the vertebral body **159** to alleviate nerve **102** impingement **184** shown in Figure 70. Figure 72 shows a longitudinal view of a fastened disc **100** by the compressor/screw **111/187** with bleeding sites **224** inflicted on both end plates **105.**

The strength of the fastened disc **100** may be greatly enhanced by healing initiated by the surgically inflicted bleeding sites **224.** Ligament reattachment to bone is a good example. A biodegradable suture rated merely for 20 pounds is used to attach a torn ligament onto a surgically inflicted bleeding bone. Within two weeks, the tensile strength of the reattached ligament can reach 50 pounds; strength increases with time. In essence, the suture is merely used to maintain the position of the torn ligament; reattachment and healing occur naturally with the surgically inflicted bleeding bone. As the bulging annulus is compressed by the compressor **111** as shown in Figure 72, adhesions form from oozing of the bleeding sites **224** between the end plate **105** and the compressed annulus. Tissue adhesion and the fastened compressor **111** work in conjunction to hold the bulging annulus in place, alleviate nerve **102** impingement **184** and allow time for the annulus to regenerate.

Similar to menisci in knees and articular cartilage in joints, the annulus has a limited capacity for healing and regeneration. For articular cartilage regeneration in the knee, an arthroscopic awl is used to create multiple holes on the articular cartilage surface, allowing blood and marrow elements to fill the defect, leading to formation of fibrocartilage. Patients have reported feeling significant improvement (Blevins F.T., et. al., Treatment of Articular Cartilage Defects in Athletes: An Analysis of Functional Outcome and Lesion Appearance, Orthopedics, Jul 21(7):761-7, 1998). No work has been done on end plate **105** puncturing to promote annular regeneration and adhesion. A qualitative in vitro investigation of adult human discs **100** showed that the end plates **105** are indeed partly permeable to solutes or nutrients. The permeation is associated with the presence of vascular contacts between the marrow spaces of the vertebral body **159** and the hyaline cartilage of the end plate **105.** One-third of the central portion and only one-tenth of the peripheral zone of the end plates **105** are available for diffusion, exchanging nutrients and waste between the disc **100** and vertebral bodies **159** (S. Holm, et. al., Nutrition of the Intervertebral Disk, Clinical Orthopaedics and Related Research, **129,** Nov-Dec:101-14, 1977). It has been suggested that nutritional deficiencies could lead to disc **100** degeneration (Nachemson A., et. al., In vitro diffusion of dye through the end plates and the annulus fibrosus of human lumbar intervertebral disks, Acta Orthop. Scand., 41:589, 1970). It has also been suggested that annular regeneration is slow due to calcified hyaline cartilage at the end plate **105** in adults, which greatly hinders transportation of nutrients. End plate **105** punctures with an awl or trocar **103** could provide passages for nutrients, leading to the acceleration of annular regeneration. Furthermore, as the disc **100** undergoes rapid repair through the open channels created in the end plate **105,** it is possible that fewer pain signals and/or shorter durations of them will be emitted from the degenerated annulus. Nerve **216** ingrowth into the disc **100** may decrease; the risks of future discogenic pain may decrease as well.

Spondylolisthesis is a condition in which a vertebral body **159** detaches and slips from a disc **100,** usually the L5 and S1 disc **100,** as shown in Figure 73. The slippage usually occurs with some erosion on the facet joint **129,** allowing the inferior articular process **143** of L5 to slip over the superior articular process **142** of S1, also shown in Figure 73. Spondylolisthesis is normally surgically treated with lumbosacral fusion using instrumentation fastened by screws vulnerable to fatigue and breakage. Instead of using instrumentation to fuse the intervertebral segments, annular adhesion and regeneration may eliminate the need of instruments and hardware. After the spine with the affected vertebral body **159** is repositioned, bleeding sites **224** are created by the trocar **103** to initiate tissue adhesion between the end-plate **105** and the disc **100,** as shown in Figure 74. A period (2-4 weeks) of low back immobilization followed by passive motion is required for proper adhesion and adequate reattachment to take place.

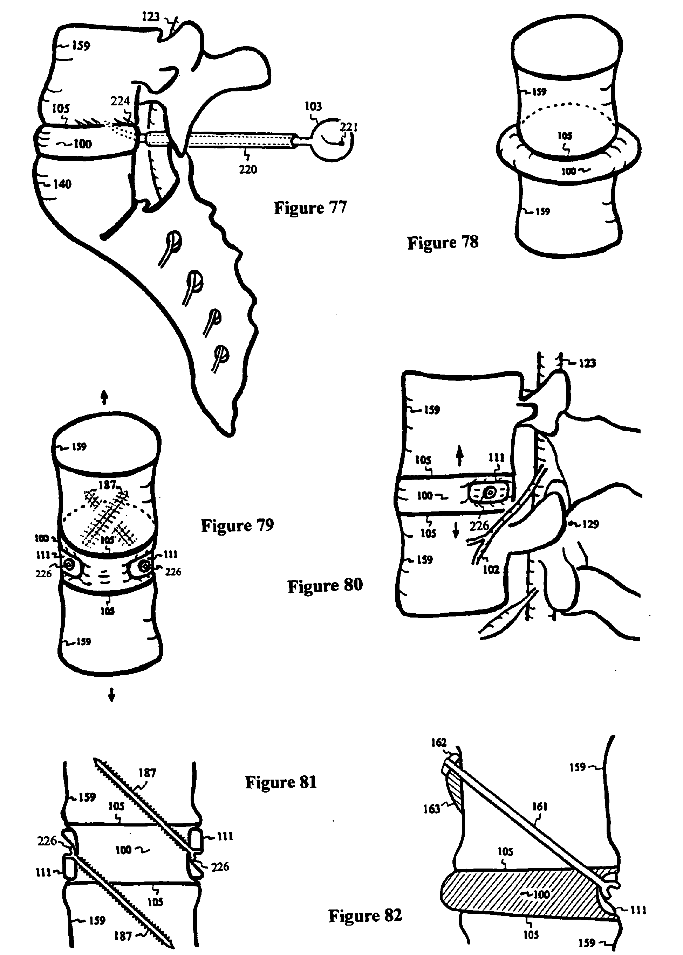

A curved trocar **103** (not part of the claimed invention but described herein to aid further understanding) made with resilient material, such as nickel-titaniun or spring tempered stainless steel, is housed in the lumen of a rigid sleeve **220,** as shown in Figure 75. The handle of the trocar **103** contains a label **221** indicating the direction of the curvature. The curved trocar **103** can be resiliently straightened within the sliding sleeve **220,** as shown in Figure 76. The curvature resumes when the sleeve **220** slides away from the curved section of the trocar **103.** The sleeve/trocar **220/103** assembly is placed perpendicular to the disc **100.** By pushing on the handle of the trocar **103,** the trocar **103** pierces through the disc **100,** resumes the unrestricted curvature and pierces into the end plate **105,** as indicated in Figure 77. The resiliently curved trocar **103** provides the surgeon greater latitude in terms of patient safety and surgically accessible locations to create bleeding sites **224** at the end plate **105.**

Figure 78 depicts a flattened or bulging disc **100** sandwiched between vertebral bodies **159,** a common cause of segmental instability and/or spinal stenosis. A pair of compressors/screws **111/187** is fastened through a portion of the disc **100,** through the end plate **105** and into the vertebral body **159,** as depicted in Figure 79. The bulging or unstable sidewall of the disc **100** is compressed, supported, fortified, stiffened, restricted, tightened, pinched in and/or fastened by the compressors/screws **111/187** to minimize segmental instability.

A pair of compressors/screws **111/187** was used to fasten a cadaveric lumbar motion segment in similar fashion as Figure 79. Motion analysis was done on the fastened cadaveric segment, showing significant increase in stability in flexion/extension and lateral bending motions. The disc height was also increased after disc **100** fastening with the compressors/screws **111/187.** The result of the cadaveric study indicates potential for treating spinal stenosis by compressing, consolidating and tucking the bulging annulus back between the vertebral bodies **159** to build disc **100** thickness and intervertebral space and to alleviate nerve **102** impingement, as shown in Figure 80. To prevent screws **187** from interfering with each other when multiple compressors **111** are used, screws **187** can be separately anchored into adjacent vertebral bodies **159,** as shown in Figure 81.

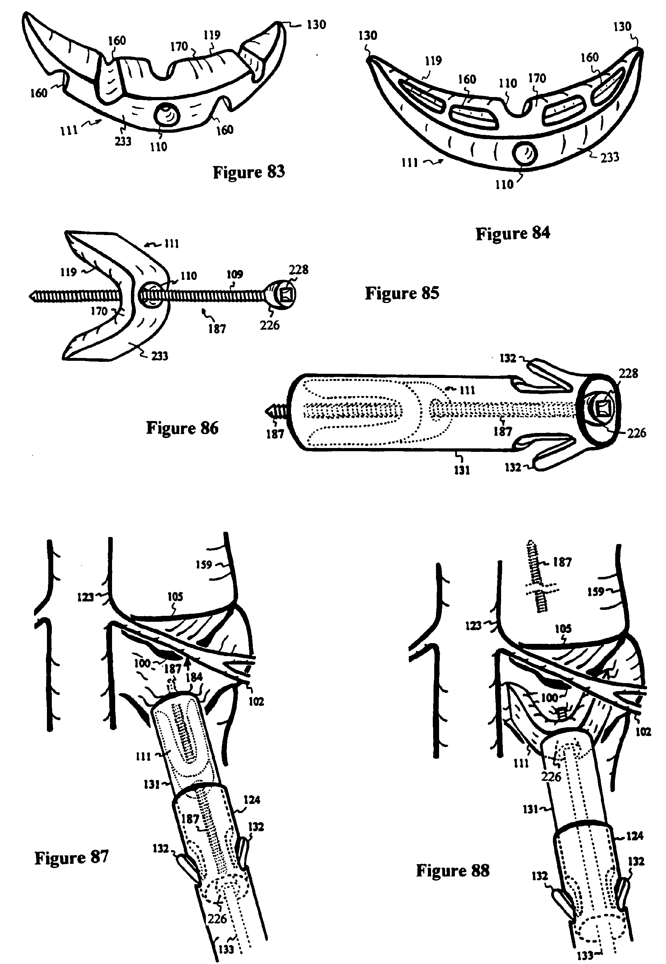

To minimize device migration, the compressor **100** can be fastened with a bolt **161** which penetrates obliquely through the vertebral body **159** and is fastened by a washer **163** and nut **162** assembly, as shown in Figure 82. Promoting tissue ingrowth into the device can also minimize device migration. Figure 83 depicts a compressor **111** with tissue ingrowth openings **160,** channels or indentations to promote annular ingrowth and prevent migration of the compressor **111.**

The compressor **111** shown in Figure 84 also indicates multiple tissue ingrowth openings **160** penetrating through the thickness of the compressor **111.** The large ingrowth openings **160** encourage annular ingrowth to prevent device migration with time. Different types of tissue ingrowth can be selected by varying the thickness of the compressor **111.** The thick compressor **111** with large ingrowth openings **160** fastened adjacent to or over the end plates **105** may encourage bone ingrowth and promote segmental fusion without removing the disc **100.** Existing spinal fusion procedure with discectomy often contributes to disc space narrowing, which may result in further nerve impingement. The segmental fusion induced by the bone ingrowth from upper and lower vertebral bodies **159** into the compressors **111** is accomplished after the distraction of the disc **100** with possible thickening of disc space. Osteoconductive material, such as bone growth factor collagen and/or hydroxyapatite, can be used to fill the tissue ingrowth openings **160.** The surfaces of the compressor **111** can also be textured or made porous, similar to hip prostheses, to promote bone ingrowth.

For discs **100** at the thoracic or cervical region, rotational motion is also significant. Figure 84 depicts a compressor **111** with tips **130** slightly curved outwardly to minimizing annular puncture during excessive or unforeseen rotations.

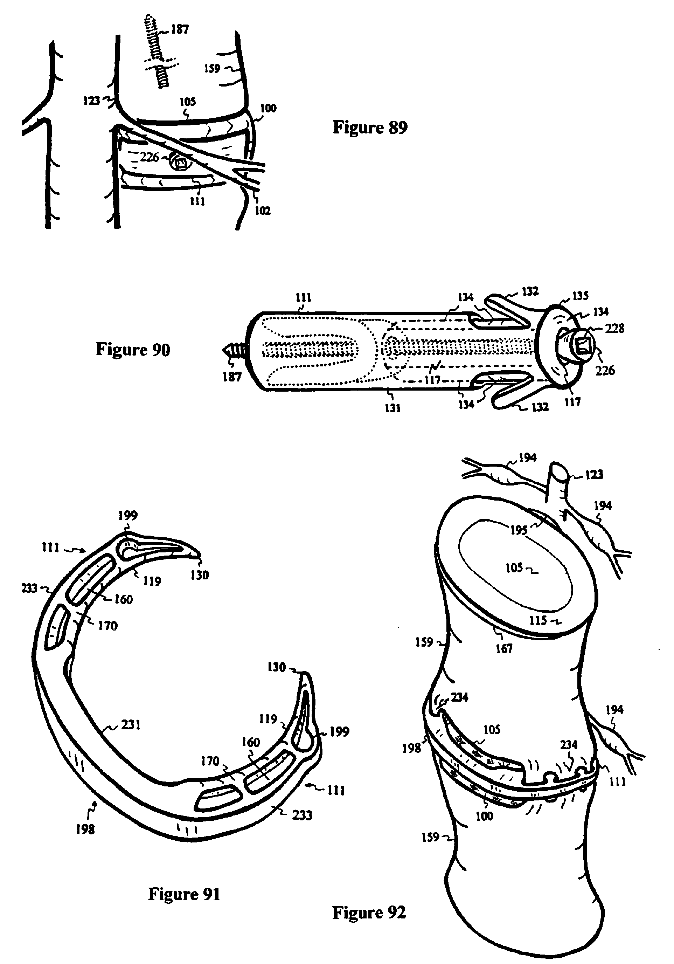

The compressor **111** can be made with a resilient or elastic material, such as nickel titanium, allowing up to 7% strain without losing shape memory. Figure 85 depicts a compressor **111** in an open or predisposed position. The resilient compressors **111** can be folded or restricted in a tubular delivery capsule **131,** as shown in Figure 86, for endoscopic insertion. In the capsule **131,** the resilient compressor **111** is in a delivery position. The delivery capsule **131** assembly holding the resilient compressor **111** and a screw **187** is fitted into a delivery device **124,** secured by latches **132** and releasable by pinching, as shown in Figure 87. The delivery device **124** is equipped with a drive **133** extending into the socket **228** opening of the screw **187.** With a small diameter or cross section of the delivery capsule **131,** it may be possible to reach the protruded disc **100** in the central zone by inserting the capsule **131** between laminae without laminotomy, as indicated in Figure 87. The screw **187** is then advanced through the disc **100** into the end plate **105.** As the screw head **226** contacts the compressor **111,** the advancing screw **187** repels the restricted compressor **111** out of the capsule **131,** as shown in Figure 88. To keep the resilient compressor **111** from rotating with the screw **187,** the cross section of the capsule **131** can be made non-circular. The repelled compressor **111** resumes the open position, spreading the legs of resilient compressors **111** on the protruded disc **100,** anterior to the nerve **102.** With further tightening of the screw **187** into the end-plate **105,** the screw head **226** presses against the compressor **111,** further spreading into a compressed position to fasten the previously bulging annulus, as shown in Figure 89.

The resilient compressor **111,** capsule **131** and screw **187** assembly is uniquely designed to accommodate the large moving range of the compressors **111** from the delivery position to the compressed position, a range even nickel-titanium alloy may not be able to provide. The uniqueness is in the open position, about half way between delivery and compressed positions. The magnitudes of the strain from the open to delivery position and from the open to compressed position are nearly equal but in opposite directions. In essence, the open or predisposed position is set at midway, making the large moving range of the compressor **111** possible, without shape memory loss.

To minimize swaying of the screw **187** during tightening, a stabilizer **134** is inserted in the capsule **131** to restrict the screw head **226** within a lumen **117** of the stabilizer **134,** as shown in Figure 90. The stabilizer **134** contains a lip **135** to prevent the stabilizer **134** from passing through the capsule **131.** As the screw head **226** in the lumen **117** advances through the disc **100,** lateral movement is greatly minimized during rotation of the socket drive **133.**

Figure 91 depicts a clamp/compressors **198/111** with large tissue ingrowth openings **160** to ensure annular ingrowth and prevent migration of the clamp/compressor **198/111.** The widening mounts **199** can also be a portion of the ingrowth openings **160.** The large ingrowth openings **160** may also allow bone ingrowth to promote spinal fusion **234** between upper and lower vertebral bodies **159,** as shown in Figure 92. The spinal fusion **234** induced by the compressors **111** can be further promoted by thick and porous compressors **111** bridging between two adjacent vertebral bodies **159,** allowing the bone from adjacent vertebral bodies **159** to grow into the ingrowth openings **160** of the compressors **111.** It is also possible to make the compressors **111** osteoconductive as hip and joint implants are, allowing bone from adjacent vertebral bodies **159** to embed and fuse with the compressors **111** and create segmental fusion **234.** The uniqueness of this spinal fusion **234** is that it is accomplished with an intact and repaired disc **100** with the possibility of increased disc height induced by disc **100** compression. Similarly, compressors **111** with osteoconductive property, porous or large ingrowth openings **160** fastened with a bracket **139,** bolt **161** or a screw **187** would provide bone ingrowth and spinal fusion **234.**

A wide range of materials can be used to fabricate the compressor **111.** Titanium, stainless steel, nickel-titanium alloy or other metallic material is preferred for strength and durability. To minimize tissue erosion, at least a portion of the compressor **111** can be made with biocompatible polymers, such as polyurethane, polypropylene, polyethylene, poly-ether-ether-ketone, acetal resin, polysulfone, polytetrafluoroethylene, polycarbonate, silicon, polyimide, ultra high molecular weight polyethylene or other. The compressor **111** can also be coated with lubricant, growth factor, nerve ingrowth inhibitor, nutrient, buffering agent, collagen, hydroxyapatite, analgesic, sealant for nucleus pulposus, blood clotting, antibiotic, radiopaque or echogenic agents. The casing **188** with pivotal peg **172,** as shown in Figure 21, can be made with stainless steel, titanium, nickel-titanium or a rigid polymer.

After the dysfunctional disc **100** has been repaired by the compressor **111,** perhaps accelerated by the surgically inflicted bleeding sites **224,** new annulus forms in a non-bulging position. Within months the strength of the repaired disc **100** may be mainly supported by the regenerated annulus cushioned between the vertebral bodies **159,** rather than from the fastening strength of the compressor **111.** Therefore, it may be possible to fabricate the compressor **111** l and the supporting devices with biodegradable material, such as poly-lactate, poly-glycolic, polycaprolactone, trimethylene carbonate, combinations of these or other materials. A biodegradable device is particularly suitable for young patients to avoid device migration or other related complications in the distant future. All materials should be able to withstand sterilization by gamma, electron beam, steam, ETO, plasma or UV light to prevent infection.

Twenty to forty percent of patients undergoing laminectomy and/or discectomy procedures do not find pain relief. Due to the high invasiveness of present procedures, epidural scarring and vertebral instability are the most common and often lingering post-surgical complications. These tissue-removing procedures are not reversible. For many patients, the pain often returns in five years or less. In contrast, the proposed compressors **111** and methods repair the dysfunctional discs **100** without tissue removal, minimizing epidural scarring and strengthening the vertebral segment. Disc compression thickens the disc **100** and distracts the adjacent vertebral bodies to alleviate pain without removing tissues and weakening the spine. The proposed devices are retrievable, and the methods do not involve with tissue removal. Discectomy, laminectomy, foraminotomy, traditional spinal fusion or other conventional procedures can be used as a fall back procedure in the event of an unsuccessful outcome.

In summary, the compressors **111** on a clamp **198,** a bracket **139,** a bolt **161** (elastic or otherwise) or a screw **187** are used for (1) compressing a protrusion to alleviate impingement, (2) fortifying the annulus to stabilize a motion segment, (3) minimizing the inward/outward bulging to protect the disc **100** from progressive delaminations, (4) atrophying the nerve to treat discogenic pain, (5) correcting the curvature of spinal deformities, (6) elevating the disc space to treat spinal stenosis, (7) sealing the leakage of nucleus pulposus to treat herniated discs **100,** and/or (8) promoting bony ingrowth to fuse the motion segment.

It is to be understood that the present invention is by no means limited to the particular constructions disclosed herein and/or shown in the drawings, but also includes any other modification, changes or equivalents within the scope of the claims. Many features have been listed with particular configurations, curvatures, options, and embodiments. The bracket **139** or the fusion plate in Figure 50 can also be viewed as the extended stop **173** of the compressor **111.** Any one or more of the features described may be added to or combined with any of the other embodiments or other standard devices to create alternate combinations and embodiments.

It should be clear to one skilled in the art that the described embodiments, materials, constructions, methods, tissues or incision sites are not the only ones which fall within the scope of the claims. Different materials, constructions, methods or designs for the compressors **111,** brackets **139** or the delivery devices **124** can be substituted and used. Nothing in the preceding description should be talcen to limit the scope of the present invention. The full scope of the invention is to be determined by the appended claims.

## Claims

1. A compression device for compressing a dysfunctional intervertebral disc (100), said compression device comprising:
an arcuate compression member (111) having a compression surface,
said compression surface having a concave curvature (170) within a horizontal plane extending therethrough,
**characterized in that** said concave curvature is sized and configured to extend at least partway around and engage the intervertebral disc, and **in that** said compression surface has a convex curvature within a vertical plane extending therethrough, and **in that** the compression device further comprises a compression means (198) for pressing said compression surface against the dysfunctional intervertebral disc (100).

2. The compression device of claim 1, wherein said convex curvature is sized and configured to fit between a patient's vertebrae.

3. The compression device of claim 1, wherein said compression member has a top surface forming a first plateau surface and a bottom surface forming a second plateau surface.

4. The compression device of claim 3, wherein at least one of said first and second plateau surfaces has an indentation; or
wherein said first and second plateau surfaces are nonparallel; or
wherein said first and second plateau surfaces are generally parallel.

5. The compression device of claim 1, wherein when viewed in said vertical plane said compression surface is generally round; or
wherein when viewed in said vertical plane said compression surface tapers to a rounded point; or
wherein when viewed in said vertical plane said compression surface is nipple-shaped; or
wherein when viewed in said vertical plane said compression surface has multiple curvatures.

6. The compression device of claim 1, further comprising a first tip and a second tip of said compression member, said first and second tips forming the ends of said concave curvature.

7. The compression device of claim 6, wherein a depth of said compression member is measured between said compression surface and an outside surface of said compression member, and wherein said depth narrows down to said first and second tips; or
wherein said tips are curved outward..

8. The compression device of claim 1, wherein an outside surface of said compression member is indented to form a depression.

9. The compression device of claim 8 wherein said depression is sized and configured to contain a portion of said compression means and, preferably, wherein said compression means is a threaded bolt and a head of said bolt is sized to fit within said depression.

10. The compression device of claim 1, further comprising a protruding leg extending from said compression member, said protruding leg sized and configured to contact a vertebra, when said compression surface is at least partially located between two vertebrae.

11. The compression device of claim 10 further comprising at least one attachment hole extending through said protruding leg; or
further comprising a second protruding leg extending from said compression member, wherein one of said protruding legs extends upwards and another of said protruding legs extends downward.

12. The compression device of claim 1, further comprising a pair of delivery tool engagement openings extending into said compression member on opposite sides thereof.

13. The compression device of claim 1, wherein said compression member is a clamp and said clamp extends around approximately three-fourths of the intervertebral disc.

14. The compression device of claim 13, wherein said compression surface is adjacent one side of the disc and further comprising a second compression surface adjacent an opposite side of the disc.

15. The compression device of claim 14, wherein said first and second compression surfaces have the same shape; or
wherein said first and second compression surfaces have different shapes; or
wherein said first and second compression surfaces are modular pieces attachable to the compression member

16. The compression device of claim 1, wherein said compression member extends around approximately one-fourth of the intervertebral disc; or
wherein said compression device is resilient; or
wherein at least a portion of said compression device is porous; or
wherein said compression device is formed of a polymer; or
wherein said compression device is formed of a nickel-titanium alloy.

17. The compression device of claim 1, wherein said compression device has at least one tissue ingrowth opening and, preferably, wherein bone can grow into said tissue ingrowth opening to form vertebrae fusion.

18. The compression device of claim 1, wherein said compression member is a resilient clamp and resiliency of said clamp provides said compression means.

19. The compression device of claim 1, wherein said compression means is a bolt.

20. The compression device of claim 19, wherein said bolt is formed of a resilient material.

21. The compression device of claim 20, wherein said bolt is biased toward a curved configuration.

22. The compression device of claim 21, wherein said curved configuration has a plurality of arcs; or
wherein said bolt has at least one slit and wherein said curved configuration is produced by the outward bowing of said slit.

23. The compression device of claim 21, further comprising a sleeve locatable around said bolt.

24. The compression device of claim 23, wherein said sleeve has a in-phase position and an out-of phase position, wherein in said out-of-phase position, said bolt is constrained in a straightened position and wherein in said in-phase position, said bolt is release into said curved configuration with at least one curve extending through an opening in said sleeve; or
wherein said sleeve is formed of a degradable material.

25. The compression device of claim 1, wherein said compression member is resilient and, preferably, further comprising a capsule, said capsule sized and configured to contain said compression member in a delivery position.

26. The compression device of claim 1, wherein said compression member is a resilient clamp and further comprising a compression member widening tool sized and configured to open and release said compression member and, preferably, wherein the compression device further comprises a first tip having a first opening therein and a second tip having a second opening therein, said first and second tips fornaing ends of said concave curvature, and wherein said compression member widening tool has two arms, each arm having a post extending therefrom, each of said posts sized and configured to engage one of said first and second openings.

## Patentansprüche

1. Druckvorrichtung zum Zusammendrücken einer dysfunktionellen Bandscheibe (100), wobei die Druckvorrichtung aufweist:
ein gebogenes Druckelement (111) mit einer Druckfläche,
wobei die Druckfläche eine konkave Krümmung (170) in einer durch sie verlaufenden horizontalen Ebene hat,
**dadurch gekennzeichnet, dass** die konkave Krümmung dimensioniert und ausgestaltet ist, um zumindest teilweise um die Bandscheibe herum zu verlaufen und an dieser anzugreifen, und dass die Druckfläche eine konvexe Krümmung in einer durch sie verlaufenden vertikalen Ebene hat und dass die Druckvorrichtung ferner ein Druckmittel (198) zum Drücken der Druckfläche gegen die dysfunktionelle Bandscheibe (100) aufweist.

2. Druckvorrichtung nach Anspruch 1, bei der die konvexe Krümmung dimensioniert und ausgestaltet ist, um zwischen die Wirbel eines Patienten zu passen.

3. Druckvorrichtung nach Anspruch 1, bei der das Druckelement eine obere Fläche, die eine erste Plateaufläche bildet, und eine untere Fläche hat, die eine zweite Plateaufläche bildet.

4. Druckvorrichtung nach Anspruch 3, bei der mindestens eine von der ersten und der zweiten Plateaufläche eine Aussparung aufweist oder
bei der die erste und die zweite Plateaufläche nicht parallel sind oder
bei der die erste und die zweite Plateaufläche im Wesentlichen parallel sind.

5. Druckvorrichtung nach Anspruch 1, bei der die Druckfläche, wenn sie in der vertikalen Ebene betrachtet wird, im Wesentlichen rund ist oder
bei der sich die Druckfläche, wenn sie in der vertikalen Ebene betrachtet wird, zu einem abgerundeten Punkt verjüngt oder
bei der die Druckfläche, wenn sie in der vertikalen Ebene betrachtet wird, nippelförmig ist oder
bei der die Druckfläche, wenn sie in der vertikalen Ebene betrachtet wird, mehrere Krümmungen hat.

6. Druckvorrichtung nach Anspruch 1, die ferner eine erste Spitze und eine zweite Spitze des Druckelements aufweist, wobei die erste und die zweite Spitze die Enden der konkaven Krümmung bilden.

7. Druckvorrichtung nach Anspruch 6, bei der eine Tiefe des Druckelements zwischen der Druckfläche und einer Außenfläche des Druckelements gemessen wird und bei der sich die Tiefe zu der ersten und der zweiten Spitze verengt oder
bei der die Spitzen nach außen gekrümmt sind.

8. Druckvorrichtung nach Anspruch 1, bei der eine Außenfläche des Druckelements eingekerbt ist, um eine Vertiefung zu bilden.

9. Druckvorrichtung nach Anspruch 8, bei der die Vertiefung dimensioniert und ausgestaltet ist, um einen Abschnitt des Druckmittels zu enthalten, und bei der bevorzugt das Druckmittel ein mit einem Gewinde versehener Bolzen ist und ein Kopf des Bolzens dimensioniert ist, um in die Vertiefung zu passen.

10. Druckvorrichtung nach Anspruch 1, die ferner ein vorstehendes Bein aufweist, das von dem Druckelement ausgeht und dimensioniert und ausgestaltet ist, um einen Wirbel zu berühren, wenn die Druckfläche zumindest teilweise zwischen zwei Wirbeln angeordnet ist.

11. Druckvorrichtung nach Anspruch 10, die ferner mindestens eine Befestigungsbohrung aufweist, die durch das vorstehende Bein verläuft, oder
die ferner ein zweites vorstehendes Bein aufweist, das von dem Druckelement ausgeht, wobei sich eines der vorstehenden Beine nach oben erstreckt und ein anderes der vorstehenden Beine nach unten erstreckt.

12. Druckvorrichtung nach Anspruch 1, die ferner ein Paar Zuführwerkzeug-Eingriffsöffnungen aufweist, die in das Druckelement an gegenüberliegenden Seiten von diesem verlaufen.

13. Druckvorrichtung nach Anspruch 1, bei der das Druckelement eine Klammer ist und die Klammer um ungefähr drei Viertel der Bandscheibe herum verläuft.

14. Druckvorrichtung nach Anspruch 13, bei der die Druckfläche benachbart zu einer Seite der Scheibe ist und die ferner eine zweite Druckfläche benachbart zu einer gegenüberliegenden Seite der Scheibe aufweist.

15. Druckvorrichtung nach Anspruch 14, bei der die erste und die zweite Druckfläche dieselbe Form haben oder
bei der die erste und die zweite Druckfläche unterschiedliche Formen haben oder
bei der die erste und die zweite Druckfläche modulare Teile sind, die an dem Druckelement befestigt werden können.

16. Druckvorrichtung nach Anspruch 1, bei der das Druckelement um ungefähr ein Viertel der Bandscheibe herum verläuft oder bei der die Druckvorrichtung elastisch ist oder
bei der zumindest ein Teil der Druckvorrichtung porös ist oder
bei der die Druckvorrichtung aus einem Polymer ausgebildet ist oder
bei der die Druckvorrichtung aus einer Nickel-TitanLegierung ausgebildet ist.

17. Druckvorrichtung nach Anspruch 1, bei der die Druckvorrichtung mindestens eine Gewebeeinwachsöffnung hat und bei der bevorzugt Knochen in die Gewebeeinwachsöffnung einwachsen kann, um eine Wirbelverbindung zu bilden.

18. Druckvorrichtung nach Anspruch 1, bei der das Druckelement eine elastische Klammer ist und die Elastizität der Klammer das Druckmittel bereitstellt.

19. Druckvorrichtung nach Anspruch 1, bei der das Druckmittel ein Bolzen ist.

20. Druckvorrichtung nach Anspruch 19, bei der der Bolzen aus einem elastischen Material ausgebildet ist.

21. Druckvorrichtung nach Anspruch 20, bei der der Bolzen zu einer gekrümmten Gestaltung hin vorgespannt ist.

22. Druckvorrichtung nach Anspruch 21, bei der die gekrümmte Gestaltung eine Vielzahl von Bogen aufweist oder bei der der Bolzen mindestens einen Schlitz aufweist und bei der die gekrümmte Gestaltung durch das sich nach außen Biegen des Schlitzes erzeugt wird.

23. Druckvorrichtung nach Anspruch 21, die ferner eine Hülse aufweist, die um den Bolzen herum angeordnet werden kann.

24. Druckvorrichtung nach Anspruch 23, bei der die Hülse eine Position in Phase und eine Position außer Phase hat, wobei der Bolzen in der Position außer Phase in einer gestreckten Stellung eingespannt ist und wobei der Bolzen in der Position in Phase in die gekrümmte Gestaltung freigegeben ist, wobei sich mindestens eine Krümmung durch eine Öffnung in der Hülse erstreckt, oder
bei der die Hülse aus einem zersetzbaren Material ausgebildet ist.

25. Druckvorrichtung nach Anspruch 1, bei der das Druckelement elastisch ist und die bevorzugt ferner eine Kapsel aufweist, die dimensioniert und ausgestaltet ist, um das Druckelement in einer Zuführposition zu enthalten.

26. Druckvorrichtung nach Anspruch 1, bei der das Druckelement eine elastische Klammer ist und die ferner ein Druckelement-Aufweitungswerkzeug aufweist, das dimensioniert und ausgestaltet ist, um das Druckelement zu öffnen und freizugeben, und bei der bevorzugt die Druckvorrichtung ferner eine erste Spitze mit einer ersten Öffnung in dieser und eine zweite Spitze mit einer zweiten Öffnung in dieser aufweist, wobei die erste und die zweite Spitze Enden der konkaven Krümmung bilden, und bei der das Druckelement-Aufweitungswerkzeug zwei Arme hat, von denen jeder einen von ihm ausgehenden Stab aufweist, wobei jeder der Stäbe dimensioniert und ausgestaltet ist, um an einer der ersten und der zweiten Öffnungen anzugreifen.

## Revendications

1. Dispositif de compression pour comprimer un disque intervertébral dysfonctionnel (100), ledit dispositif de compression comprenant :
- un élément de compression (111) en forme d'arc ayant une surface de compression,
- ladite surface de compression ayant une courbure concave (170) dans un plan horizontal s'étendant à travers celle-ci,
**caractérisé par le fait que** ladite courbure concave est dimensionnée et configurée pour s'étendre au moins en partie autour du et engager le disque intervertébral, et **par le fait que** ladite surface de compression a une courbure convexe dans un plan vertical s'étendant à travers celle-ci, et **par le fait que** le dispositif de compression comprend en outre un moyen de compression (198) pour presser ladite surface de compression contre le disque intervertébral dysfonctionnel (100).

2. Dispositif de compression selon la revendication 1, dans lequel ladite courbure convexe est dimensionnée et configurée pour s'adapter entre les vertèbres d'un patient.

3. Dispositif de compression selon la revendication 1, dans lequel ledit élément de compression a une surface supérieure formant une première surface de plateau et une surface inférieure formant une seconde surface de plateau.

4. Dispositif de compression selon la revendication 3, dans lequel au moins l'une desdites première et seconde surfaces de plateau a une entaille ; ou
dans lequel lesdites première et seconde surfaces de plateau sont non parallèles ; ou
dans lequel lesdites première et seconde surfaces de plateau sont généralement parallèles.

5. Dispositif de compression selon la revendication 1, dans lequel, lorsqu'elle est observée dans ledit plan vertical, ladite surface de compression est généralement arrondie ; ou
dans lequel, lorsqu'elle est observée dans ledit plan vertical, ladite surface de compression s'effile jusqu'à un point arrondi ; ou
dans lequel, lorsqu'elle est observée dans ledit plan vertical, ladite surface de compression est en forme de mamelon ; ou
dans lequel, lorsqu'elle est observée dans ledit plan vertical, ladite surface de compression des courbures multiples.

6. Dispositif de compression selon la revendication 1, comprenant en outre une première extrémité et une seconde extrémité dudit élément de compression, lesdites première et seconde extrémités formant les extrémités de ladite courbure concave.

7. Dispositif de compression selon la revendication 6, dans lequel une profondeur dudit élément de compression est mesurée entre ladite surface de compression et une surface extérieure dudit élément de compression, et dans lequel ladite profondeur se rétrécit jusqu'auxdites première et seconde extrémités ; ou dans lequel lesdites extrémités sont courbées vers l'extérieur.

8. Dispositif de compression selon la revendication 1, dans lequel une surface extérieure dudit élément de compression est entaillée pour former une dépression.

9. Dispositif de compression selon la revendication 8, dans lequel ladite dépression est dimensionnée et configurée pour contenir une partie dudit moyen de compression et, de préférence, dans lequel ledit moyen de compression est un boulon fileté et une tête dudit boulon est dimensionnée pour s'adapter à l'intérieur de ladite dépression.

10. Dispositif de compression selon la revendication 1, comprenant en outre une jambe en saillie s'étendant à partir dudit élément de compression, ladite jambe en saillie étant dimensionnée et configurée pour entrer en contact avec une vertèbre, lorsque ladite surface de compression est au moins partiellement située entre deux vertèbres.

11. Dispositif de compression selon la revendication 10, comprenant en outre au moins un trou de fixation s'étendant à travers ladite jambe en saillie ; ou comprenant en outre une seconde jambe en saillie s'étendant à partir dudit élément de compression, dans lequel l'une desdites jambes en saillie s'étend vers le haut et l'autre desdites jambes en saillie s'étend vers le bas.

12. Dispositif de compression selon la revendication 1, comprenant en outre une paire d'ouvertures d'engagement d'outils d'introduction s'étendant dans ledit élément de compression sur des côtés opposés de celui-ci.

13. Dispositif de compression selon la revendication 1, dans lequel ledit élément de compression est une bride de serrage et ladite bride de serrage s'étend approximativement autour des trois quarts du disque intervertébral.

14. Dispositif de compression selon la revendication 13, dans lequel ladite surface de compression est adjacente à un côté du disque et comprenant en outre une seconde surface de compression adjacente à un côté opposé du disque.

15. Dispositif de compression selon la revendication 14, dans lequel lesdites première et seconde surfaces de compression ont la même forme ; ou
dans lequel lesdites première et seconde surfaces de compression ont des formes différentes ; ou
dans lequel lesdites première et seconde surfaces de compression sont des pièces modulaires attachables à l'élément de compression.

16. Dispositif de compression selon la revendication 1, dans lequel ledit élément de compression s'étend approximativement autour d'un quart du disque intervertébral ; ou
dans lequel ledit dispositif de compression est élastique ; ou
dans lequel au moins une partie dudit dispositif de compression est poreuse ; ou
dans lequel ledit dispositif de compression est formé d'un polymère ; ou
dans lequel ledit dispositif de compression est formé d'un alliage nickel-titane.

17. Dispositif de compression selon la revendication 1, dans lequel ledit dispositif de compression a au moins une ouverture d'interposition de tissu et, de préférence, dans lequel l'os peut se développer à l'intérieur de l'ouverture d'interposition de tissu pour former une fusion des vertèbres.

18. Dispositif de compression selon la revendication 1, dans lequel ledit élément de compression est une bride de serrage élastique et l'élasticité de ladite bride de serrage fournit ledit moyen de compression.

19. Dispositif de compression selon la revendication 1, dans lequel ledit moyen de compression est un boulon.

20. Dispositif de compression selon la revendication 19, dans lequel ledit boulon est formé d'une matière élastique.

21. Dispositif de compression selon la revendication 20, dans lequel ledit boulon est sollicité vers une configuration incurvée.

22. Dispositif de compression selon la revendication 21, dans lequel ladite configuration incurvée a une pluralité d'arcs ; ou
dans lequel ledit boulon a au moins une fente et dans lequel ladite configuration incurvée est produite par la incurvation vers l'extérieur de ladite fente.

23. Dispositif de compression selon la revendication 21, comprenant en outre un manchon positionnable autour dudit boulon.

24. Dispositif de compression selon la revendication 23, dans lequel ledit manchon a une position en phase et une position en décalage de phase, dans lequel dans ladite position en décalage de phase, ledit boulon est contraint dans une position rectiligne et dans lequel, dans ladite position en phase, ledit boulon est libéré dans ladite configuration incurvée avec au moins une courbe s'étendant à travers une ouverture dans ledit manchon ; ou
dans lequel ledit manchon est formé d'une matière dégradable.

25. Dispositif de compression selon la revendication 1, dans lequel ledit élément de compression est élastique et, de préférence, comprenant en outre une capsule, ladite capsule étant dimensionnée et configurée pour contenir ledit élément de compression dans une position d'introduction.

26. Dispositif de compression selon la revendication 1, dans lequel ledit élément de compression est une bride de serrage élastique et comprenant en outre un outil d'élargissement de l'élément de compression, dimensionné et configuré pour ouvrir et libérer ledit élément de compression et, de préférence, dans lequel le dispositif de compression comprend en outre une première extrémité ayant une première ouverture dans celle-ci et une seconde extrémité ayant une seconde ouverture dans celle-ci, lesdites première et seconde extrémités formant des extrémités de ladite courbure concave, et dans lequel ledit outil d'élargissement de l'élément de compression a deux bras, chaque bras ayant une colonne s'étendant à partir de celui-ci, chacune desdites colonnes étant dimensionnée et configurée pour engager l'une desdites première et seconde ouvertures.
